# EUROPEAN PATENT SPECIFICATION

(11) **EP 4 013 434 B1**
(45) Date of publication and mention of the grant of the patent: **23.10.2024**
(21) Application number: 20764587.0
(22) Date of filing: 14.08.2020
(51) Int. Cl.: A61K 35/74, A61P 3/10

(54) **BACTERIUM COMPRISING MYO-INOSITOL TO PROPIONATE PATHWAY**
BAKTERIUM MIT MYO-INOSITOL-ZU-PROPIONAT-SIGNALWEG
BACTÉRIE COMPRENANT UNE VOIE MYO-INOSITOL VERS PROPIONATE

(30) Priority: 14.08.2019 NL 2023645; 24.12.2019 NL 2024569
(43) Date of publication of application: 22.06.2022
(73) Proprietor: Wageningen Universiteit, 6708 PB Wageningen (NL)
(72) Inventor: DE VOS, Willem Meindert, 1082 PB AMSTERDAM (NL); BUI, Thi Phuong Nam, 6721 PK BENNEKOM (NL)
(74) Representative: EP&C
(86) International application number: PCT/EP2020/072925
(87) International publication number: WO 2021/028585

(56) References cited:
- WO-A1-2018/187272
- T. P. N. BUI ET AL: "Anaerostipes rhamnosivorans sp. nov., a human intestinal, butyrate-forming bacterium", INTERNATIONAL JOURNAL OF SYSTEMATIC AND EVOLUTIONARY MICROBIOLOGY, vol. 64, no. Pt 3, 11 November 2013 (2013-11-11), GB, pages 787 - 793, XP055227328, ISSN: 1466-5026, DOI: 10.1099/ijs.0.055061-0

## Description

### Technical field

The present invention relates to the field of preventing and/or treating metabolic diseases, such as metabolic syndrome and insulin resistance or insulin resistance-related conditions, including dyslipidemia and type 2 diabetes mellitus as well as insulin-resistance in endocrine diseases (e.g., obese subjects with type 1 diabetes mellitus, Cushing's disease or lipodystrophy syndromes).

### Background of the disclosure

Insulin resistance is a medical condition wherein cells are resistant to the insulin, leading to a high blood sugar level. Patients with type 2 diabetes mellitus usually have passed through an earlier stage of insulin resistance, although this earlier stage often goes undiagnosed.

Dietary supplementation of myo-inositol has been found to be efficient in lowering postprandial plasma glucose in several animal models of diabetes or insulin resistance (Ortmeyer et al 1993 Endocrinology 132: 646-651; Ortmeyer et al 1995 Obesity Research 3: 605S-608S; Croze and Soulage 2013 Biochimie 95: 1811-1827).

Subjects with type 2 diabetes mellitus have been shown to display decreased inositol phosphoglycan levels in muscle biopsies as compared to healthy controls, and myo-inositol has been proposed as putative downstream second messenger of the insulin signalling pathway (Kennington et al 1990 New England Journal of Medicine 323: 373-378).

Dietary myo-inositol can be derived from phospholipids or phytic acid (a plant component present in beans, seed and nuts), from which it is liberated by phytases. Moreover, the human body can produce myo-inositol from glucose.

However, although it is known that dietary myo-inositol supplementation can reduce insulin resistance, the mechanism through which this occurs is unknown.

It is an objective of the present disclosure to provide a new or improved strategy for preventing and/or treating metabolic diseases, in particular metabolic syndrome and insulin resistance or insulin resistance-related conditions, including dyslipidemia and type 2 diabetes mellitus as well as insulin-resistance in endocrine diseases (e.g., obese subjects with type 1 diabetes mellitus, Cushing's disease or lipodystrophy syndromes).

### Summary of the disclosure

The invention is as set out in the claims.

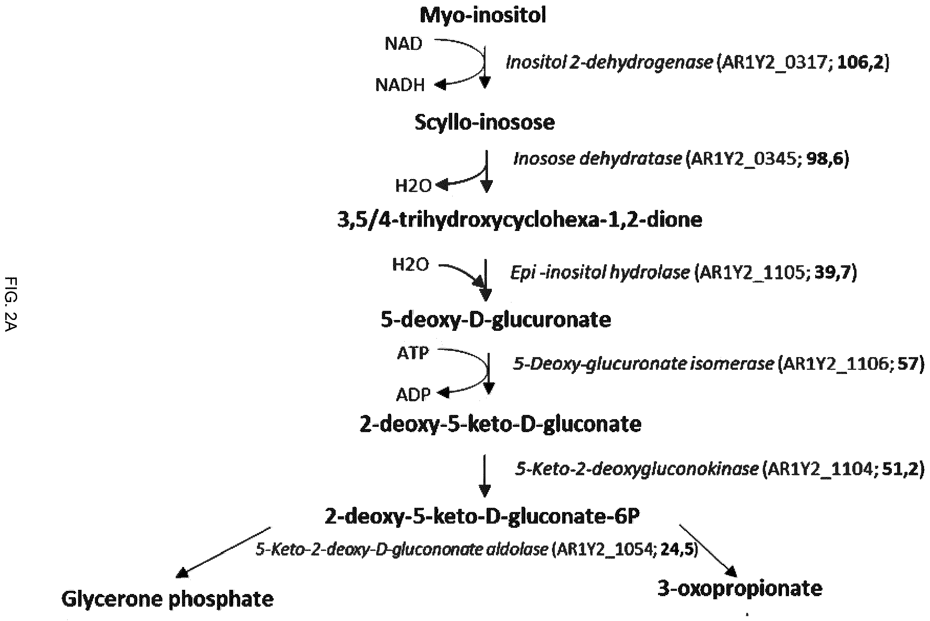

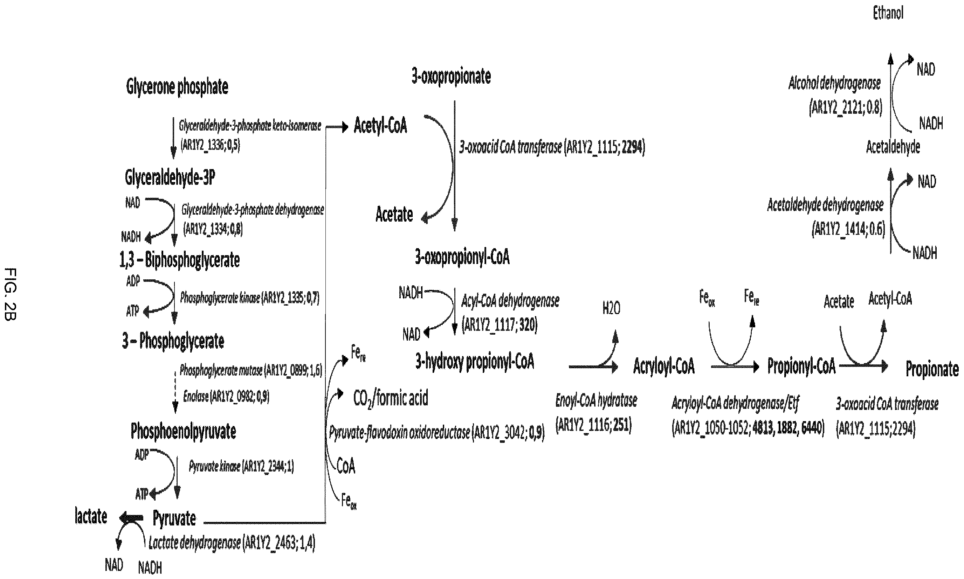

The present inventors found that myo-inositol can serve as carbon and energy source for a butyrogenic bacterium *Anaerostipes rhamnosivorans* that was isolated from stool of a healthy infant (Bui et al 2014 International Journal of Systematic and Evolutionary Microbiology 64: 787-793). This bacterium is able to ferment efficiently myo-inositol to propionic acid and a complete inositol fermentation pathway has been found in its closed genome, and which optionally can be introduced in any other bacterium. See Figure 2A and 2B.

It is known that dietary myo-inositol supplementation can reduce insulin resistance. However, the mechanism through which this occurs was unknown before the present invention was made.

The present inventors considered that myo-inositol may have an indirect beneficial effect, and that it is the metabolic conversion of myo-inositol that is beneficial to the subject, rather than the myo-inositol itself. Hence, it is of interest to note that the production of butyrate and, notably, propionate by *A. rhamnosivorans* is certainly beneficial for the subject (see e.g. Hamer et al 2008 Pharmacology & Therapeutics 27: 104-119; Hosseini et al 2011 Nutrition Reviews 69: 245-258).

The present inventors envisage supplementation of a subject with a bacterium according to the present disclosure, or alternatively both myo-inositol and a bacterium according to the present disclosure, to provide for a direct or synergistic effect leading to improved insulin resistance via *in situ* propionic acid and optionally butyric acid production.

### Detailed description of the disclosure

The present disclosure relates to a bacterium comprising a gene set encoding an inositol to propionic acid pathway that allows said bacterium to convert inositol to propionic acid or a salt or ester thereof, for use in preventing and/or treating metabolic syndrome, insulin resistance and/or insulin resistance related condition.

Specifically, the bacterium is capable of converting inositol into propionic acid or a derivative thereof via inositol metabolism and propionic acid synthesis. The inositol may be chiro-inositol or myo-inositol, preferably myo-inositol. In a preferred embodiment, the bacterium additionally is a butyric acid and/or butyrate-producing (butyrogenic) bacterium (i.e. also comprises a butyric acid / butyrate synthesis pathway gene set).

Without wishing to be bound by any theories, it is believed that the bacterium according to the present disclosure (or strains derived therefrom), when administered to a human being or when ingested by a human being in an adequate amount, is able to survive and at least transiently colonize the GI tract of said human being. This colonization enables greater *in situ* production of propionic acid / propionate (and/or butyric acid / butyrate). Increased *in situ* production of propionic acid is believed to underlie the beneficial effects as taught herein, e.g. preventing and/or treating conditions or diseases such as metabolic diseases, such as metabolic syndrome and insulin resistance or insulin resistance-related complications, such as dyslipidemia and type 2 diabetes mellitus as well as insulin-resistance in endocrine diseases (e.g.,obese subjects with type 1 diabetes mellitus, Cushing's disease or lipodystrophy syndromes).

### Bacterium

In a first aspect, the present disclosure relates to a bacterium comprising a gene set encoding an inositol to propionic acid pathway that allows said bacterium to convert inositol to propionic acid or a salt or ester thereof, in particular but not necessarily under anaerobic conditions, such as wherein the bacterium and/or its medium is not in contact with gas comprising more than 1, 2, 3, 4, 5, 10 vol% oxygen. The bacterial strain is preferably, but not necessarily, an isolate, e.g. a human intestinal isolate.

The inositol to propionic acid pathway gene set may comprise one or more (at least two or three, or all) of the genes encoding the proteins: myo-inositol 2-dehydrogenase, inosose dehydratase, epi-inositol hydrolase, 5-deoxy-glucuronate isomerase, 5-keto-2-deoxygluconokinase, 5-keto-2-deoxy-D-gluconate-6 phosphate aldolase, glyceraldehyde-3-phosphate keto-isomerase, glyceraldehyde-3-phosphate dehydrogenase, phosphoglycerate kinase, phosphoglycerate mutase, enolase, pyruvate kinase, pyruvate-flavodoxin oxidoreductase, 3-oxoacid CoA transferase. acyl-CoA dehydrogenase, enoyl-CoA hydratase, and acryloyl-CoA dehydrogenase/Etf. Additionally, the inositol to propionic acid pathway gene set may comprise the gene encoding the protein inosose isomerase and/or another copy of the enzyme myo-inositol 2-dehydrogenase since it allows for conversion of D-chiro-inositol to scyllo-inosose.

Additionally or alternatively, the bacterium according to the present disclosure may comprise a gene encoding lactate dehydrogenase, a gene encoding acetaldehyde dehydrogenase and/or a gene encoding alcohol dehydrogenase.

In a preferred embodiment, at least one (or at least two or three or all) of the proteins: myo-inositol 2-dehydrogenase, inosose dehydratase, epi-inositol hydrolase, 5-deoxy-glucuronate isomerase, 5-keto-2-deoxygluconokinase, 5-keto-2-deoxy-D-gluconate-6 phosphate aldolase, and glyceraldehyde-3-phosphate keto-isomerase, glyceraldehyde-3-phosphate dehydrogenase, phosphoglycerate kinase, phosphoglycerate mutase, enolase, pyruvate kinase, pyruvate-flavodoxin oxidoreductase, 3-oxoacid CoA transferase, acyl-CoA dehydrogenase, enoyl-CoA hydratase, acryloyl-CoA dehydrogenase/Etf and inosose isomerase, may be overproduced (or the genes encoding therefor overexpressed) when the bacterium is grown in the presence of (myo) inositol as compared to when the bacterium is grown in the absence of (myo) inositol.

In the context of the present disclosure, the amount of (myo) inositol used to determine whether genes are upregulated or proteins are overexpressed in a bacterium as compared to when said bacterium is grown in absence of (myo) inositol may be in the range of from about 5 mM to about 100 mM, preferably from about 10 mM to about 50 mM, more preferably from about 15 mM to about 25 mM, and is most preferably about 20 mM.

In an embodiment, the bacterium according to the present disclosure may be an isolated intestinal bacterial strain, or strain derived therefrom, and/or may be an intestinal bacterium isolated from a human being, which naturally comprises an inositol to propionic acid pathway gene set as taught herein and which is capable of converting (myo and/or chiro) inositol to propionic acid or a derivative thereof.

In a further aspect, the bacterium according to the disclosure may be the bacterium *Anaerostipes rhamnosivorans* 1y2 deposited by Wageningen University on June 26, 2015 at the Centraalbureau voor Schimmelcultures (now named Westerdijk Fungal Biodiversity Institute) located at Uppsalalaan 8, 3584CT Utrecht, the Netherlands, assigned the deposit number CBS 140182. Also encompassed is any bacterial strain derived from the deposited bacterium.

Alternatively, the bacterium according to the present disclosure may be a bacterium which has been transfected with the inositol to propionic acid pathway gene set as taught herein, and which is capable of converting (myo and/or chiro) inositol to propionic acid or a derivative thereof. The skilled person is well-acquainted with methods for transforming bacteria with a desired genetic construct (i.e. pathway gene set).

In an embodiment, the bacterium according to the present disclosure belongs to the phylum *Firmicutes*, preferably to the class Clostridia (and/or order Clostridiales), more preferably to the family Lachnospiraceae, more preferably to the genus *Anaerostipes*, even more preferably to the species *Anaerostipes rhamnosivorans.*

In an embodiment, the bacterium according to the present disclosure is not *Anaerostipes hadrus.* Alternatively, in case the bacterium according to the present disclosure is *Anaerostipes hadrus*, the use is for preventing and/or treating insulin resistance or insulin resistance related complication as taught herein. Preferably the *Anaerostipes hadrus* concerns type strain DSM3319 or 108065. Alternatively, the bacterium according to the present disclosure is *Anaerostipes butyraticus*, preferably type strain DSM22094.

As set out herein before, the inositol to propionic acid pathway gene set may comprise one or more of the genes encoding the proteins: myo-inositol 2-dehydrogenase, inosose dehydratase, epi-inositol hydrolase, 5-deoxy-glucuronate isomerase, 5-keto-2-deoxygluconokinase, 5-keto-2-deoxy-D-gluconate-6 phosphate aldolase, glyceraldehyde-3-phosphate keto-isomerase, glyceraldehyde-3-phosphate dehydrogenase, phosphoglycerate kinase, phosphoglycerate mutase, enolase, pyruvate kinase, pyruvate-flavodoxin oxidoreductase, 3-oxoacid CoA transferase, acyl-CoA dehydrogenase, enoyl-CoA hydratase, and acryloyl-CoA dehydrogenase/Etf. Additionally, the inositol to propionic acid pathway gene set may comprise the gene encoding the protein inosose isomerase and/or another copy of the enzyme myo-inositol 2-dehydrogenase since each will allow for conversion of D-chiro-inositol to scyllo-inosose.

The gene encoding myo-inositol 2-dehydrogenase may have at least 70, 80, 90, 95, 99, 100% sequence identity with SEQ ID NO:1. Additionally or alternatively the gene encoding myo-inositol 2-dehydrogenase may concern a myo-inositol 2-dehydrogenase that has at least 70, 80, 90, 95, 99, 100% sequence identity with SEQ ID NO:2. The function of the gene myo-inositol 2-dehydrogenase can be seen in the conversion of myo-inositol to scyllo-inosose.

A further or alternative copy of the gene encoding myo-inositol 2-dehydrogenase may have at least 70, 80, 90, 95, 99, 100% sequence identity with SEQ ID NO:3. Additionally or alternatively, the further or alternative copy of the gene encoding myo-inositol 2-dehydrogenase may concern a myo-inositol 2-dehydrogenase that has at least 70, 80, 90, 95, 99, 100% sequence identity with SEQ ID NO:4. The function of this gene can be seen in a conversion from D-chiro-Inositol to 1-keto-D-chiro-Inositol.

The gene encoding inosose dehydratase may have at least 70, 80, 90, 95, 99, 100% sequence identity with SEQ ID NO:5. Additionally or alternatively the gene encoding inosose dehydratase may concern an inosose dehydratase that has at least 70, 80, 90, 95, 99, 100% sequence identity with SEQ ID NO: 6. The function of the gene inosose dehydratase can be seen in the conversion of scyllo-inosose to 3,5/4-trihydroxycyclohexa-1,2-dione.

The gene encoding epi-inositol hydrolase may have at least 70, 80, 90, 95, 99, 100% sequence identity with SEQ ID NO:7. Additionally or alternatively the gene encoding epi-inositol hydrolase may concern an epi-inositol hydrolase that has at least 70, 80, 90, 95, 99, 100% sequence identity with SEQ ID NO:8. The function of the gene epi-inositol hydrolase can be seen in the conversion of 3,5/4-trihydroxycyclohexa-1,2-dione to 5-deoxy-D-glucuronate.

The gene encoding 5-deoxy-glucuronate isomerase may have at least 70, 80, 90, 95, 99, 100% sequence identity with SEQ ID NO:9. Additionally or alternatively the gene encoding 5-deoxy-glucuronate isomerase may concern a 5-deoxy-glucuronate isomerase that has at least 70, 80, 90, 95, 99, 100% sequence identity with SEQ ID NO:10. The function of the gene 5-deoxy-glucuronate isomerase can be seen in the conversion of 5-deoxy-D-glucuronate to 2-deoxy-5-keto-D-gluconate.

The gene encoding 5-keto-2-deoxygluconokinase may have at least 70, 80, 90, 95, 99, 100% sequence identity with SEQ ID NO:11. Additionally or alternatively the gene encoding 5-keto-2-deoxygluconokinase may concern a 5-keto-2-deoxygluconokinase that has at least 70, 80, 90, 95, 99, 100% sequence identity with SEQ ID NO:12. The function of the gene 5-keto-2-deoxygluconokinase can be seen in the conversion of 2-deoxy-5-keto-D-gluconate to 2-deoxy-5-keto-D-gl uconate-6P.

The gene encoding 5-keto-2-deoxy-D-gluconate-6 phosphate aldolase may have at least 70, 80, 90, 95, 99, 100% sequence identity with SEQ ID NO:13. Additionally or alternatively the gene encoding 5-keto-2-deoxy-D-gluconate-6 phosphate aldolase may concern a 5-keto-2-deoxy-D-gluconate-6 phosphate aldolase that has at least 70, 80, 90, 95, 99, 100% sequence identity with SEQ ID NO: 14. The function of the gene 5-keto-2-deoxy-D-gluconate-6 phosphate aldolase can be seen in the conversion of 2-deoxy-5-keto-D-gluconate-6P to 3-oxopropionate and glycerone phosphate.

The gene encoding glyceraldehyde-3-phosphate keto-isomerase may have at least 70, 80, 90, 95, 99, 100% sequence identity with SEQ ID NO:15. Additionally or alternatively the gene encoding glyceraldehyde-3-phosphate keto-isomerase may concern a glyceraldehyde-3-phosphate keto-isomerase that has at least 70, 80, 90, 95, 99, 100% sequence identity with SEQ ID NO:16. The function of the gene glyceraldehyde-3-phosphate keto-isomerase can be seen in the conversion of glycerone phosphate to glyceraldehyde-3P.

The gene encoding glyceraldehyde-3-phosphate dehydrogenase may have at least 70, 80, 90, 95, 99, 100% sequence identity with SEQ ID NO:17. Additionally or alternatively the gene encoding glyceraldehyde-3-phosphate dehydrogenase may concern a glyceraldehyde-3-phosphate dehydrogenase that has at least 70, 80, 90, 95, 99, 100% sequence identity with SEQ ID NO:18. The function of the gene glyceraldehyde-3-phosphate dehydrogenase can be seen in the conversion of glyceraldehyde-3P to 1,3-biphosphoglycerate.

The gene encoding phosphoglycerate kinase may have at least 70, 80, 90, 95, 99, 100% sequence identity with SEQ ID NO:19. Additionally or alternatively the gene encoding phosphoglycerate kinase may concern a phosphoglycerate kinase that has at least 70, 80, 90, 95, 99, 100% sequence identity with SEQ ID NO:20. The function of the gene phosphoglycerate kinase can be seen in the conversion of 1,3-biphosphoglycerate to 3-phosphoglycerate.

The gene encoding phosphoglycerate mutase may have at least 70, 80, 90, 95, 99, 100% sequence identity with SEQ ID NO:21. Additionally or alternatively the gene encoding phosphoglycerate mutase may concern a phosphoglycerate mutase that has at least 70, 80, 90, 95, 99, 100% sequence identity with SEQ ID NO:22. The function of the gene phosphoglycerate mutase can be seen in the conversion of 3-phosphoglycerate to 2-phosphoenolpyruvate.

The gene encoding enolase may have at least 70, 80, 90, 95, 99, 100% sequence identity with SEQ ID NO:23. Additionally or alternatively the gene encoding enolase may concern a enolase that has at least 70, 80, 90, 95, 99, 100% sequence identity with SEQ ID NO:24. The function of the gene enolase can be seen in the conversion of 3-phosphoglycerate to 2-phosphoenolpyruvate.

The gene encoding pyruvate kinase may have at least 70, 80, 90, 95, 99, 100% sequence identity with SEQ ID NO:47. Additionally or alternatively the gene encoding pyruvate kinase may concern a pyruvate kinase that has at least 70, 80, 90, 95, 99, 100% sequence identity with SEQ ID NO:48. The function of the gene pyruvate kinase can be seen in the conversion of phosphoenolpyruvate to pyruvate.

The gene encoding pyruvate-flavodoxin oxidoreductase may have at least 70, 80, 90, 95, 99, 100% sequence identity with SEQ ID NO:25. Additionally or alternatively the gene encoding pyruvate-flavodoxin oxidoreductase may concern a pyruvate-flavodoxin oxidoreductase that has at least 70, 80, 90, 95, 99, 100% sequence identity with SEQ ID NO:26. The function of the gene pyruvate-flavodoxin oxidoreductase can be seen in the conversion of pyruvate to acetyl-CoA and CO₂/formic acid.

The gene encoding 3-oxoacid CoA transferase may have at least 70, 80, 90, 95, 99, 100% sequence identity with SEQ ID NO:27. Additionally or alternatively the gene encoding 3-oxoacid CoA transferase may concern a 3-oxoacid CoA transferase that has at least 70, 80, 90, 95, 99, 100% sequence identity with SEQ ID NO:28. The function of the gene 3-oxoacid CoA transferase can be seen in the conversion of 3-oxopropionate to 3-oxopropionyl-CoA and/or in the conversion of propionyl-CoA to propionate.

The gene encoding acyl-CoA dehydrogenase may have at least 70, 80, 90, 95, 99, 100% sequence identity with SEQ ID NO:29. Additionally or alternatively the gene encoding acyl-CoA dehydrogenase may concern a acyl-CoA dehydrogenase that has at least 70, 80, 90, 95, 99, 100% sequence identity with SEQ ID NO:30. The function of the gene acyl-CoA dehydrogenase can be seen in the conversion of 3-oxopropionyl-CoA to 3-hydroxy propionyl-CoA.

The gene encoding enoyl-CoA hydratase may have at least 70, 80, 90, 95, 99, 100% sequence identity with SEQ ID NO:31. Additionally or alternatively the gene encoding enoyl-CoA hydratase may concern a enoyl-CoA hydratase that has at least 70, 80, 90, 95, 99, 100% sequence identity with SEQ ID NO:32. The function of the gene enoyl-CoA hydratase can be seen in the conversion of 3-hydroxy propionyl-CoA to acryloyl-CoA.

The gene encoding acryloyl-CoA dehydrogenase/Etf may have at least 70, 80, 90, 95, 99, 100% sequence identity with SEQ ID NO:33. A further (second) or alternative copy of the gene encoding acryloyl-CoA dehydrogenase/Etf may have at least 70, 80, 90, 95, 99, 100% sequence identity with SEQ ID NO:35. A further (third) or alternative copy of the gene encoding acryloyl-CoA dehydrogenase/Etf may have at least 70, 80, 90, 95, 99, 100% sequence identity with SEQ ID NO:37. Additionally or alternatively the gene encoding acryloyl-CoA dehydrogenase/Etf may concern a acryloyl-CoA dehydrogenase/Etf that has at least 70, 80, 90, 95, 99, 100% sequence identity with SEQ ID NO:34. A further (second) or alternative copy of the gene encoding acryloyl-CoA dehydrogenase/Etf may concern a acryloyl-CoA dehydrogenase/Etf that has at least 70, 80, 90, 95, 99, 100% sequence identity with SEQ ID NO:36. A further (third) or alternative copy of the gene encoding acryloyl-CoA dehydrogenase/Etf may concern a acryloyl-CoA dehydrogenase/Etf that has at least 70, 80, 90, 95, 99, 100% sequence identity with SEQ ID NO:38. Acryloyl-CoA dehydrogenase/Eft can be a complex in which AR1Y2_1051 and AR1Y2_1052 are the alpha and beta subunit of a complex that function as the electron acceptor of the acryolyl-CoA dehydrogenase (AR1Y2_1050). The function of the gene acryloyl-CoA dehydrogenase/Etf can be seen in the conversion of acryloyl-CoA to propionyl-CoA.

The gene encoding inosose isomerase may have at least 70, 80, 90, 95, 99, 100% sequence identity with SEQ ID NO:39. Additionally or alternatively the gene encoding inosose isomerase may concern a inosose isomerase that has at least 70, 80, 90, 95, 99, 100% sequence identity with SEQ ID NO:40. The function of this gene can be seen in a conversion from 1-keto-D-chiro-Inositol to scyllo-inosose.

The gene encoding lactate dehydrogenase may have at least 70, 80, 90, 95, 99, 100% sequence identity with SEQ ID NO:41. Additionally or alternatively the gene lactate dehydrogenase may concern a lactate dehydrogenase that has at least 70, 80, 90, 95, 99, 100% sequence identity with SEQ ID NO:42. The function of the gene lactate dehydrogenase can be seen in the conversion of pyruvate to lactate.

The gene encoding acetaldehyde dehydrogenase may have at least 70, 80, 90, 95, 99, 100% sequence identity with SEQ ID NO:43. Additionally or alternatively the gene acetaldehyde dehydrogenase may concern an acetaldehyde dehydrogenase that has at least 70, 80, 90, 95, 99, 100% sequence identity with SEQ ID NO:44. The function of the gene acetaldehyde dehydrogenase can be seen in the conversion of acetyl-CoA to acetaldehyde.

The gene encoding alcohol dehydrogenase may have at least 70, 80, 90, 95, 99, 100% sequence identity with SEQ ID NO:45. Additionally or alternatively the gene alcohol dehydrogenase may concern an alcohol dehydrogenase that has at least 70, 80, 90, 95, 99, 100% sequence identity with SEQ ID NO:46. The function of the gene alcohol dehydrogenase can be seen in the conversion of acetaldehyde to ethanol.

In certain embodiments, the bacterium according to the present disclosure does not comprise a gene encoding malonate-semialdehyde dehydrogenase (involved in the conversion of malonic semi-aldehyde to acetyl-CoA).

### Compositions

The present disclosure further provides a composition comprising a bacterium according to the present disclosure and optionally a physiologically acceptable carrier. The physiologically acceptable carrier may be any carrier that is suitable for keeping the bacterium as taught herein viable until consumption by a subject (e.g. human or animal). For instance, non-limiting examples of acceptable carriers that are suitable for this purpose include any of well-known physiological or pharmaceutical carriers, buffers, and excipients. It will be appreciated that the choice for a suitable physiological or pharmaceutical carrier will depend upon the intended mode of administration of the composition as taught herein (e.g. oral) and the intended form of the composition (e.g. beverage, yogurt, powder, capsules, and the like). The skilled person knows how to select a physiological or pharmaceutical carrier, which is suitable for the compositions as taught herein.

In an embodiment, the composition as taught herein may be in the form of a food composition, feed composition, feed supplement composition, food supplement composition or pharmaceutical composition. The composition is preferably suitable for consumption by a human being.

In an embodiment, the composition is a food or food supplement composition. The food or food supplement composition may be selected from the group consisting of a liquid, liquid beverage (including dairy beverage and fermented beverage), yogurt, cheese, gel, gelatine, gelatine capsule, powder, paste, pressed tablet, and gel cap. In a suitable embodiment, the composition is a liquid, preferably a liquid beverage (e.g. dairy beverage). The food or food supplement composition may be a dairy product, preferably a fermented dairy product, preferably a yogurt or a yogurt drink.

In an embodiment, the composition as taught herein may be a probiotic composition. Such probiotic composition may comprise the (isolated) bacterium as taught herein, or a strain derived therefrom. In an embodiment, the composition as taught herein further comprises one or more additional beneficial isolated intestinal bacterial strain.

In one embodiment, the one or more additional beneficial isolated intestinal bacterial strain may be any lactic acid bacterial strain selected from the genera *Lactobacillus* and/or *Bifidobacterium* and/or any butyrate-producing bacteria which produces butyrate via the acetyl-CoA pathway.

In an embodiment, the composition may be a symbiotic composition. It may be advantageous to add one or more prebiotic ingredients to the composition as taught herein, for example, to supplement the effects (e.g. production of propionic acid/propionate and/or butyric acid / butyrate or a derivative thereof) of the bacterium as taught herein.

In an embodiment, the one or more prebiotic ingredients may be any prebiotic ingredients, which are suitable to enhance the activity and/or stimulate the growth of the bacterium, or a strain derived therefrom, as taught herein. Non-limiting examples of suitable prebiotic ingredients include fibres such as inulin, pectin, and resistant starch, as well as cellobiose, maltose, mannose, salicine, trehalose, amygdalin, arabinose, melibiose, sorbitol, rhamnose and/or xylose.

In an embodiment, the composition as taught herein comprises an (myo)inositol-rich source and/or (myo) inositol. For instance, it may be advantageous to add a (myo)inositol rich source and/or (myo) inositol to the composition as taught herein to further promote the production of propionic acid/propionate and/or butyric acid / butyrate or a derivative thereof in the GI tract of a mammal (e.g. human being).

In an embodiment, the composition as taught herein may comprise one or more ingredients which are suitable for promoting survival and/or viability of the bacterium or strain derived therefrom as taught herein during storage and/or during exposure to bile and/or during passage through the GI tract of a mammal (e.g. a human being). Non-limiting examples of such ingredients include an enteric coating, and controlled release agents allowing passage through the stomach. The skilled person knows how to select suitable ingredients for maintaining a bacterium as taught herein viable and functional i.e. able to carry out intended function(s).

In one embodiment, the compositions as taught herein may further comprise one or more ingredients, which further enhance the nutritional value and/or the therapeutic value the compositions as taught herein. For instance, it may be advantageous to add one or more ingredients (e.g. nutritional ingredients, veterinary or medicinal agents etc.) selected from proteins, amino acids, enzymes, mineral salts, vitamins (e.g. thiamine HCl, riboflavin, pyridoxine HCl, niacin, inositol, choline chloride, calcium pantothenate, biotin, folic acid, ascorbic acid, vitamin B12, p-aminobenzoic acid, vitamin A acetate, vitamin K, vitamin D, vitamin E, and the like), sugars and complex carbohydrates (e.g. water-soluble and water-insoluble monosaccharides, disaccharides, and polysaccharides), medicinal compounds (e.g. antibiotics), antioxidants, trace element ingredients (e.g. compounds of cobalt, copper, manganese, iron, zinc, tin, nickel, chromium, molybdenum, iodine, chlorine, silicon, vanadium, selenium, calcium, magnesium, sodium and potassium and the like). The skilled person is familiar with methods and ingredients that are suitable to enhance the nutritional and/or therapeutic/medicinal value of the compositions as taught herein.

The bacterium as taught herein may be incorporated into the composition in lyophilized form, microencapsulated form (reviewed by, for example, Solanki et al. BioMed Res. Int. 2013, Article ID 620719), or any other form preserving the activity and/or viability of the bacterial strain.

The composition as taught herein may be a pharmaceutical composition. The pharmaceutical composition may be for use as a supplement. A pharmaceutical composition will usually comprise a pharmaceutical carrier, in addition to the bacterium taught herein. The carrier is preferably an inert carrier. The preferred form depends on the intended mode of administration and (therapeutic) application. A pharmaceutical carrier can be any compatible, nontoxic substance suitable to deliver the bacterium taught herein to the GI tract of a subject. For example, sterile water, or inert solids may be used as a carrier, usually complemented with a pharmaceutically acceptable adjuvant, buffering agent, dispersing agent, and the like. A pharmaceutical composition as taught herein may be in liquid form, e.g. a stabilized suspension of bacteria of the bacterium taught herein, or in solid form, e.g., a powder of lyophilized bacteria taught herein. In case the bacterium taught herein is lyophilized, a cryoprotectant such as lactose, trehalose or glycogen can be employed. E.g., for oral administration, the bacterium taught herein can be administered in solid dosage forms, such as capsules, tablets, and powders, comprising lyophilized bacteria, or in liquid dosage forms, such as elixirs, syrups, and suspensions. The bacterium taught herein, e.g., in lyophilized form, can be encapsulated in capsules such as gelatin capsules, together with inactive ingredients and powdered carriers, such as e.g. glucose, lactose, sucrose, mannitol, starch, cellulose or cellulose derivatives, magnesium stearate, stearic acid, sodium saccharin, talcum, magnesium carbonate and the like.

In an embodiment, the bacterium as taught herein may be comprised in the composition as taught herein in an amount ranging from about 10⁶ to about 10¹⁵ colony forming units (CFU). For instance, the intestinal bacteria may be comprised in the composition in an amount of about 10⁷ CFU to about 10¹⁴ CFU, preferably about 10⁸ CFU to about 10¹³ CFU, preferably about 10⁹ CFU to about 10¹² CFU, more preferably about 10¹⁰ CFU to about 10¹² CFU.

### Methods and uses of the disclosure

The present disclosure is concerned with a bacterium as taught herein or a composition as taught herein for use as a medicament, for use as a food or food supplement, or for use as a probiotic and/or symbiotic.

In a preferred embodiment, the bacterium for use according to the present disclosure may be administered separately, sequentially or simultaneously with (myo) inositol, or with a (myo) inositol source (or a phospholipid or phytic acid source) which may be present in the same composition as the bacterium, or in a separate composition, for example comprising in the range of from about 5 mM to about 100 mM, preferably from about 10 mM to about 50 mM, more preferably from about 15 mM to about 25 mM, and is most preferably about 20 mM (myo)inositol, or for example comprising from about 1 mg to about 1000 mg, preferably from about 10 mg to about 500 mg, more preferably from about 15 mg to about 50 mg. Alternatively, the amount of inositol and/or administration frequency is chosen such to that between 0.1 and 100 grams is consumed per day, or between 0.5 and 10, or between 0.5 and 10, or between 0.1 and 5, all in grams per day.

The present disclosure particularly pertains to the bacterium as taught herein or a composition as taught herein for use in maintaining, restoring and/or improving GI health in general, and/or for preventing and/or treating conditions or diseases such as obesity, metabolic diseases, such as metabolic syndrome and insulin resistance or insulin resistance-related complications, such as dyslipidemia and type 2 diabetes mellitus as well as insulin-resistance in endocrine diseases (e.g.,obese subjects with type 1 diabetes mellitus, Cushing's disease or lipodystrophy syndromes).

The present disclosure is also directed to a method for maintaining, restoring and/or improving GI health in general, and/or for preventing and/or treating conditions or diseases such as obesity, metabolic diseases, such as metabolic syndrome and insulin resistance or insulin resistance-related complications, such as dyslipidemia and type 2 diabetes mellitus as well as insulin-resistance in endocrine diseases (e.g.,obese subjects with type 1 diabetes mellitus, Cushing's disease or lipodystrophy syndromes) in a subject in need thereof, said method comprising the step of increasing the level of the bacterium as taught herein in said subject.

The level of the bacterium as taught herein in said subject may be increased by administering an effective amount of said bacterium to said subject, and/or by administering an effective amount of a compound capable of increasing the level of said bacterium in (the GI tract of) said subject.

In an embodiment, the bacterium as taught herein may be administered concomitant with (myo)inositol or a (myo)inositol source (or a phospholipid or phytic acid source), such as fruits, beans, grains, and nuts. The skilled person can, without undue burden, readily identify (myo)inositol-rich sources.

In a preferred embodiment, the level of the bacterium as taught herein in the GI tract of a subject may be increased by administering an effective amount of the bacterium as taught herein, but preferably *Anaerostipes rhamnosivorans* 1y2 and/or a composition as taught herein comprising *Anaerostipes rhamnosivorans* 1y2, to said subject.

In an embodiment, the subject may be selected from the group consisting of human beings, non-human primates, mice, rats, dogs, cows, and pigs. In a preferred embodiment, the subject is a human.

The disclosure also relates to a method for producing propionic acid, said method comprising the step of contacting the bacterium as taught herein with a suitable energy source, e.g. (myo)inositol and/or glucose/acetate, under conditions which allow the bacterium as taught herein to convert the energy source to propionic acid.

The methods taught herein may be in vitro methods.

### GENERAL DEFINITIONS

The term 'probiotics' or 'probiotic products' as used herein refers to microorganisms such as intestinal bacteria, which - when administered or ingested in effective amounts - confer health benefits to the host (e.g. humans or mammals). Preferably, probiotics should be alive or viable when administered to a subject so as to allow the probiotics to colonize the large intestine of the host. However, under certain conditions, probiotics may also be dead when administered provided that substances produced by the probiotics still exert probiotic, beneficial effects on the host. Most probiotics or probiotic products are composed of lactic acid bacteria such as *Lactobacilli* or *Bifidobacteria.* The skilled person is well-acquainted with the field of probiotics and knows how to select lactic acid bacteria endowed with probiotic activity.

The term 'prebiotics' or 'prebiotic products' as used herein generally refers to compounds that promote the growth and/or activity of GI microorganisms that contribute to the well-being of their host. Prebiotics or prebiotic products consist mainly of fermentable fibres or non-digestible carbohydrates. The fermentation of these fibres by probiotics promotes the production of beneficial end products, such as SCFAs, particularly butyrate. The skilled person is well-acquainted with the field of prebiotics and knows how to select ingredients endowed with prebiotic activity.

The term 'symbiotics' or 'symbiotic products' as used herein generally refers to compositions and/or nutritional supplements combining probiotics and one or more compounds that promote the growth and/or activity of GI microorganisms, such as prebiotics, into one product. The symbiotic beneficially affects the host by improving the survival and colonization of the probiotic in the GI tract, by selectively stimulating the growth and/or by activating the metabolism of the probiotic, thus improving host welfare. The skilled person is well-acquainted with symbiotics and knows how to select ingredients that may be combined into a symbiotic.

The term 'propionic acid' as used herein refers is a carboxylic acid with chemical formula CH₃CH₂CO₂H. The term may include derivatives thereof, i.e. compounds derived from propionic acid and in particular salts and esters of propionic acid which are known as propionates or propanoates. Non-limiting examples of propionate salts are ammonium propionate, calcium propionate, magnesium propionate, potassium propionate and sodium propionate. Non-limiting example of propionate ester is ethyl propionate.

The term 'butyric acid' (also known under the systematic name butanoic acid) as used herein refers to a carboxylic acid with the structural formula CH₃CH₂CH₂COOH. The term may include derivatives thereof, i.e. compounds derived from butyric acid and includes salts and esters of butyric acid, which are known as butyrate or butanoate. Non-limiting examples of butyrate salts include sodium butyrate, calcium butyrate, magnesium butyrate, manganese butyrate, cobalt butyrate, barium butyrate, lithium butyrate, zinc butyrate, potassium butyrate, ferrous butyrate and the like. Non-limiting examples of butyrate esters (i.e. esters of butyric acid) include cellulose acetate butyrate, methyl butyrate, ethyl butyrate, butyl butyrate, pentyl butyrate, and the like.

The terms 'butyrate-producing bacterium' or 'butyric acid-producing bacterium' or 'butyrogenic bacterium' are used interchangeably herein and refer to a bacterium which is capable of producing butyric acid and/or butyrate and/or one or more derivatives thereof. A prominent pathway by which butyric acid and/or butyrate and derivative thereof may be produced *in situ* in the mammalian gut (or *in vitro* in culture), i.e. a butyric acid/butyrate synthesis pathway gene set, is the so-called 'acetyl-CoA pathway'. The acetyl-CoA pathway has been well-documented and is known to be particularly prevalent in intestinal bacteria belonging, for instance, to the genus *Lachnospiraceae* and *Ruminococcaceae* (which together may form up to 20% of total gut microbiota). According to the acetyl-CoA pathway, butyric acid and/or butyrate and/or derivatives thereof may be formed by a single bacterial species via carbohydrate fermentation and/or by a group of microorganisms where metabolites from other organisms act as a substrate for butyrogenic bacteria. The conventional acetyl-CoA pathway involves a cascade of enzymes, including (among many others) two key enzymes referred to as butyryl-CoA transferase (But) and butyrate kinase (Buk). The skilled person is well-acquainted with the 'acetyl-CoA pathway' including genes coding for enzymes and other elements underlying the functioning of said pathway as well as intestinal bacterial species that have this pathway.

The term 'inositol to propionic acid (or propionate) pathway gene set' as used herein refers to a set of genes that encode proteins involved in the conversion of (myo) inositol into propionic acid (propionate) or a derivative thereof. In an embodiment of the present disclosure, the pathway gene set comprises genes encoding the proteins: myo-inositol 2-dehydrogenase, inosose dehydratase, epi-inositol hydrolase, 5-deoxy-glucuronate isomerase, 5-keto-2-deoxygluconokinase, 5-keto-2-deoxy-D-gluconate-6 phosphate aldolase, glyceraldehyde-3-phosphate keto-isomerase, glyceraldehyde-3-phosphate dehydrogenase, phosphoglycerate kinase, phosphoglycerate mutase, enolase, pyruvate kinase, pyruvate-flavodoxin oxidoreductase, 3-oxoacid CoA transferase. acyl-CoA dehydrogenase, enoyl-CoA hydratase, acryloyl-CoA dehydrogenase/Etf and optionally inosose isomerase and/or a second copy of myo-inositol 2-dehydrogenase.

The term 'beneficial intestinal bacteria species' as used herein refers to a bacterium species that inhabits (i.e. is innate) the mammalian (e.g. human) intestine and exerts beneficial effect(s) (e.g. protection against pathogenic bacteria species, production of butyric acid and/or butyrate and derivatives, etc.) on the GI, metabolic and other health of a mammal in which it resides. Non-limiting examples of beneficial intestinal bacterial species include lactic acid bacteria from the genera *Lactobacillus* and *Bifidobacterium.* Other non-limiting examples of beneficial intestinal bacterial species include butyrate-producing bacterial species, which use the acetyl-CoA to produce butyric acid and/or butyrate and derivative thereof, such as the bacterial strains disclosed in US2014/0242654, WO 2014/150094 or WO2013032328 A1. Pathogenic bacterial species may refer to a bacterium that inhabits (i.e. is innate) the mammalian (e.g. human) intestine and exerts deleterious effect(s) (e.g. infection) on the GI health of a mammal in which it resides. A notorious non-limiting example of a pathogenic bacterial species is the toxin-producing *Clostridium difficile.*

The term `effective amount' as used herein refers to an amount necessary to achieve an effect as taught herein. For instance, an effective amount of the bacterium as taught herein is an amount which is effectively useful for maintaining, restoring, and/or improving GI heath in a human being, for converting (myo)inositol into propionic acid/propionate or a derivative thereof and/or butyric acid/ butyrate or a derivative thereof and/or for preventing and/or treating conditions or diseases described herein, in a subject, preferably a human being. These conditions or diseases include, without limitation, obesity, metabolic diseases, such as metabolic syndrome and insulin resistance or insulin resistance-related complications, such as dyslipidemia and type 2 diabetes mellitus as well as insulin-resistance in endocrine diseases (e.g.,obese subjects with type 1 diabetes mellitus, Cushing's disease or lipodystrophy syndromes). The effective amount can be readily determined without undue experimentation by a person of ordinary skill in the art.

The term 'a strain that derives therefrom' as used herein relates to strains obtained by using the deposited strain as taught herein as starting material. The strain that derives therefrom may be a mutant strain, which may be derived from a strain of the invention by means of, for instance, genetic engineering, radiation, UV light, chemical treatment. Alternatively, such derivative or mutant strain may be a strain derived from the deposited strain as taught herein that has been subjected to growth adaptation to particular conditions resulting in an additional benefit to the derivative strain, such as more rapid growth, better survival in the gut, enhanced inositol to propionate conversion, using methods that are well-known to the skilled person. It is preferred that the derivative or mutant is functionally equivalent to the deposited strain as taught herein. A preferred derivative or mutant as taught herein has substantially the same activity or function as the deposited strain as taught herein, i.e. has the ability to convert (myo)inositol to propionic acid/propionate and derivatives. The derivative or mutant advantageously provides substantially the same benefits to a mammal (e.g. humans or other mammals) administered with said derivative or mutant as would be the case upon administration of the deposited strain. The derivative or mutant strain may also be a spontaneous derivative or mutant strain having the same characteristics as described herein for the deposited strain.

The term 'suitable for consumption' or `nutritionally acceptable' refers to ingredients or substances, which are generally regarded as safe for human (as well as other mammals) consumption.

The term 'metabolic syndrome' is well-known by the skilled person. Within the present disclosure, the term encompasses all conditions diagnosed as "metabolic syndrome" by an (authorized) medical practitioner. For example, metabolic syndrome may be diagnosed if a patient has at least two, or at least three of the following traits:
- Large waist - A waistline that measures at least 35 inches (89 centimeters) for women and 40 inches (102 centimeters) for men;
- High triglyceride level - 150 milligrams per deciliter (mg/dL), or 1.7 millimoles per liter (mmol/L), or higher of this type of fat found in blood;
- Reduced "good" or HDL cholesterol - Less than 40 mg/dL (1.04 mmol/L) in men or less than 50 mg/dL (1.3 mmol/L) in women of high-density lipoprotein (HDL) cholesterol;
- Increased blood pressure - 130/85 millimeters of mercury (mm Hg) or higher;
- Elevated fasting blood sugar - 100 mg/dL (5.6 mmol/L) or higher.

The term 'insulin resistance' is well known by the skilled person. Within the present disclosure, the term encompasses all conditions diagnosed as "insulin resistance" by an (authorized) medical practitioner. For example, insulin resistance may be diagnosed by the gold standard for determining and quantifying insulin resistance which is the "hyperinsulinemic euglycemic clamp" ( DeFronzo RA, Tobin JD, Andres R 1979). The American Journal of Physiology. 237 (3): E214-23). This method measures the amount of glucose necessary to compensate for an increased insulin level without causing hypoglycemia. The procedure may take about two hours and typically involves the following steps. Through a peripheral vein, insulin is infused at 10-120 mU per m² per minute. In order to compensate for the insulin infusion, glucose 20% is infused to maintain blood sugar levels between 5 and 5.5 mmol/L. The rate of glucose infusion is determined by checking the blood sugar levels every five to ten minutes (Muniyappa R, Lee S, Chen H, Quon MJ (January 2008). American Journal of Physiology. Endocrinology and Metabolism. 294 (1): E15-26). The rate of glucose infusion during the last thirty minutes of the test determines insulin sensitivity. If high levels (7.5 mg/min or higher) are required, the patient is insulin-sensitive. Low levels (4.0 mg/min or lower) indicate insulin resistance. Levels between 4.0 and 7.5 mg/min are not definitive, and suggest "impaired glucose tolerance," an early sign of insulin resistance. Alternatively, the homeostatic model assessment (HOMA) is a method used to determine and quantify insulin resistance, as it correlates reasonably with the golden standard. See e.g. Matthews DR, Hosker JP, Rudenski AS, Naylor BA, Treacher DF, Turner RC (1985). Diabetologia. 28 (7): 412-9. doi:10.1007/BF00280883. PMID 3899825; and/or A. S. Rudenski; D. R. Matthews; J. C. Levy; R. C. Turner (1991) Metabolism. 40 (9): 908-917. A HOMA(-IR) score that deviates from a reference range can indicate insulin resistance. Alternatively, a fasting serum insulin level greater than 25 mU/L or 174 pmol/L can be considered as indicating insulin resistance. The term "insulin resistance" as used herein refers to peripheral insulin resistance and/or hepatic insulin resistance. The term 'insulin resistance-related conditions' (or complications) may refer to dyslipidemia. Alternatively or additionally, the term may refer to type 2 diabetes mellitus. Further, the term 'insulin resistance-related conditions' (or complications) may refer to insulin-resistance in endocrine disease (e.g.,obese subjects with type 1 diabetes mellitus, Cushing's disease or lipodystrophy syndromes).

The term 'about', as used herein indicates a range of normal tolerance in the art, for example within 2 standard deviations of the mean. The term 'about' can be understood as encompassing values that deviate at most 10%, 9%, 8%, 7%, 6%, 5%, 4%, 3%, 2%, 1%, 0.5%, 0.1%, 0.05%, or 0.01% of the indicated value.

The terms 'comprising' or 'to comprise' and their conjugations, as used herein, refer to a situation wherein said terms are used in their non-limiting sense to mean that items following the word are included, but items not specifically mentioned are not excluded. It also encompasses the more limiting verb 'to consist essentially of' and 'to consist of'.

Reference to an element by the indefinite article 'a' or 'an' does not exclude the possibility that more than one of the elements is present, unless the context clearly requires that there be one and only one of the elements. The indefinite article 'a' or 'an' thus usually means 'at least one'.

The terms 'to increase' and 'increased level' and the terms 'to decrease' and 'decreased level' refer to the ability to significantly increase or significantly decrease or to a significantly increased level or significantly decreased level. Generally, a level is increased or decreased when it is at least 5%, such as 10%, 15%, 20%, 25%, 30%, 35%, 40%, 45%, 50% higher or lower, respectively, than the corresponding level in a control or reference. Alternatively, a level in a sample may be increased or decreased when it is statistically significantly increased or decreased compared to a level in a control or reference.

### Brief description of the figures

**Figure 1****.** A bacterium according to the present disclosure is able to ferment efficiently myo-inositol to propionate. The experiment was performed twice.
**Figures 2A****/****2B****.** the identified inositol to propionate pathway.

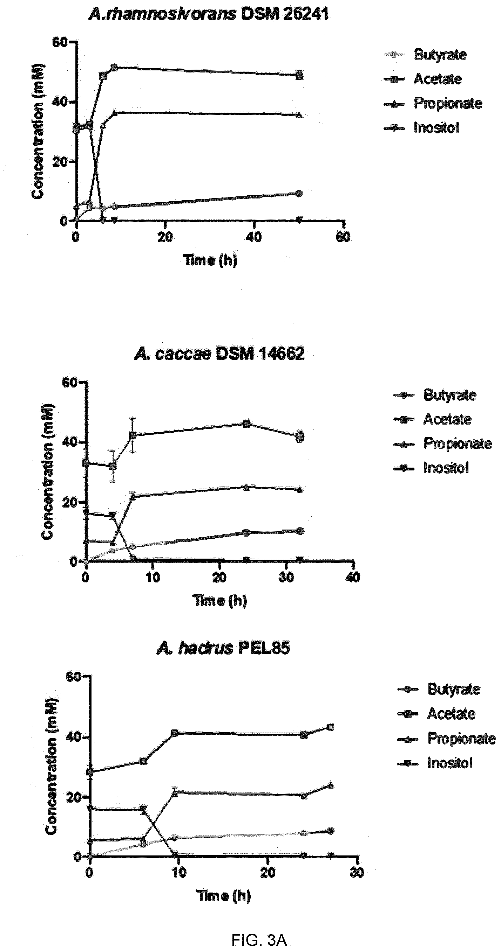

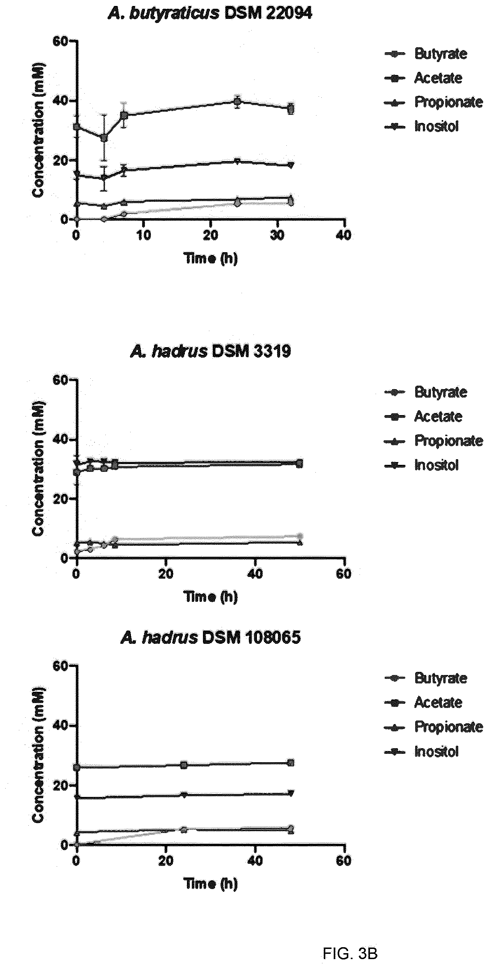

**Figure 3A****/****3B****.** shows the capabilities of metabolizing myo-inositol by different Anaerostipes strains

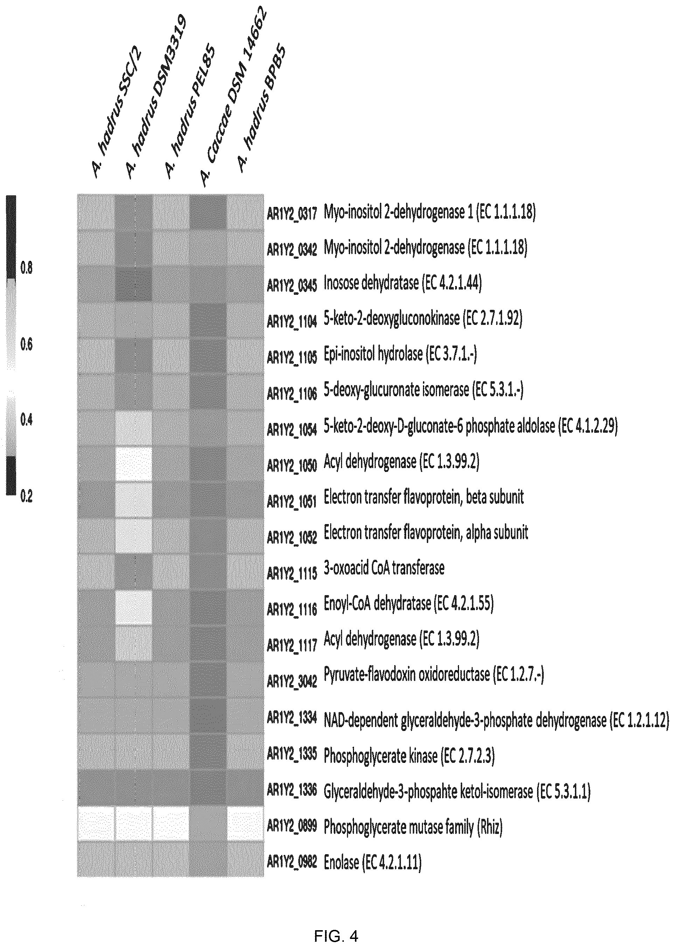

**Figure 4****.** the presence of inositol pathway genes in NCBI genomes of Anaerostipes isolates

### Sequence listing

| ***Myo-inositol 2-dehydrogenase 1* - *AR1Y2_0317*** |
|---|
| Nucleotide sequence (SEQ ID NO:1) |
| atggtaaatgtaggtattatcggagcaggaagaatcggaagagtacatgtagaaagcatctgcacacaggtaggaaacgca aaggttaagactcttgctgatccttttatgtcagaggacacagcagtatgggcaaaagaaatgggtgttgaaaataccaccaaa gattacaaagacatcttaaatgatcctcagatcgacgcagtgctgatctgctcatccacagatacacattcaccgatctccgtag aggctattgaagcaggaaaacatgtattctgcgaaaagccaatcgaccatgacgtacagaagattcaggaagtagtcgacg ctttaaagaaaagcgacgtaaaataccaggtaggattcaaccgccgttttgaccataactttgaagccatccgggatgccgtgg ctgctggaaagattggtgagacacagatcatcaaagttacatccagagatcctgaaccgccaagtgctgattatgtaaaagttt ccggaggaatgttcctggacatgacaatccatgattttgacatggtccgtttccttgcaggatgtgacgctgaagaagtgtatgta gaagctgcagtccttgtagatcctgccatcggtgaagccggagatgtggacactgctgttatcacattaaaaatgaaaaacggt tccatcgccgtgattgacaactcaagaaaagccgtttacggatacgatcagagagctgaagtgttcggatcaaagggaatggt cgtaacagcaaacgattccgcatcctcagccgtgatcagtaatgcggacggagtgacaggagaaaaacctctttacttcttcct ggaacgctacatggcagcctatgcaaaagaaatcaaatgctttatcgacgccatcgaaaaaaacacagacacccctcttgg agttatggacggtcttgagccagtacttatggggcttgcggctaaaaaatcattagaagaacaccgcccagtaaaaatttctga aattacagtatag |
| Amino acid sequence (SEQ ID NO:2) |
| MVNVGIIGAGRIGRVHVESICTQVGNAKVKTLADPFMSEDTAVWAKEMGVENTTKDYKDIL NDPQIDAVLICSSTDTHSPISVEAIEAGKHVFCEKPIDHDVQKIQEVVDALKKSDVKYQVGF NRRFDHNFEAIRDAVAAGKIGETQIIKVTSRDPEPPSADYVKVSGGMFLDMTIHDFDMVRF LAGCDAEEVYVEAAVLVDPAIGEAGDVDTAVITLKMKNGSIAVIDNSRKAVYGYDQRAEVF GSKGMVVTANDSASSAVISNADGVTGEKPLYFFLERYMAAYAKEIKCFIDAIEKNTDTPLGV MDGLEPVLMGLAAKKSLEEHRPVKISEITV |

| ***Myo-inositol 2-dehydrogenase 2* - *AR1Y2_0342*** |
|---|
| Nucleotide sequence (SEQ ID NO:3) |
| atgaacaaagtaagagtaggtattgctggtttaggaagattagggaaattacatgcagagaatatcgcattcaagattccaaac gcggaacttacagcggcctgttcgatcgcgccagaggaattggaatatgccacagaacatttcggggtaaccgatgtttataca gatttttatgagatgttagataaagcagacattgatgtcgtggcggtggtgaccacgagcggagaacactgctggcagatcaaa gcagcattggacgctggaaaacatgttttttgcgataaacctctcggggttacggtcaatgaatgtaaaacagcggaggccgc ggtggaacgccacccagagcttacattttttcttggatttatgcgccgttatgacccatcctacgcttatgcgaaaaagaaaatcc aggagggagccattggtaagccgtatatggtaaaagcggcagggatcgacccggaagctctggtggagggagcagtaaaa tttgcagccaccagcggcggtatttttatcgacatggcaatccatgatatcgatttaatgcgctggcttttggaatccgatccggtgg aagtctacgctgcaggggccacgtttaaacatcccgaattcagggaggcaggggatgacgagacgggggcagccatgtac aaatgtgagaacggtgcactgggatttgtacatgttgggcgtacagcggcccatggatatcatgtggagacagagatcatcgg aacagagggaagcctgagaatcagtcctgtaccggaaaagaacctgtgtatgatctacgatgagaacggagctgtgacaga |
| atgtgtcagcggttttgcagaacggtttgcagaggcatacctgttggaaatgaaggaattcatcaactgtgtccagagcggcac aaagccggatgtgaccgtatatgacggcacaaaatccacacagatcggttttgccacaacagaagcgtggaaaaagggctg tactgcaaagatccagtactag |
| Amino acid sequence (SEQ ID NO:4) |
| MNKVRVGIAGLGRLGKLHAENIAFKIPNAELTAACSIAPEELEYATEHFGVTDVYTDFYEML DKADIDVVAVVTTSGEHCWQIKAALDAGKHVFCDKPLGVTVNECKTAEAAVERHPELTFFL GFMRRYDPSYAYAKKKIQEGAIGKPYMVKAAGIDPEALVEGAVKFAATSGGIFIDMAIHDID LMRWLLESDPVEVYAAGATFKHPEFREAGDDETGAAMYKCENGALGFVHVGRTAAHGY HVETEIIGTEGSLRISPVPEKNLCMIYDENGAVTECVSGFAERFAEAYLLEMKEFINCVQSG TKPDVTVYDGTKSTQIGFATTEAWKKGCTAKIQY |

| ***Inosose dehydratase* - *AR1Y2_0345*** |
|---|
| Nucleotide sequence (SEQ ID NO:5) |
| atgtttaacaaagaaaaagtaaaattaggaattgccccgatcgcttggaccaacgatgatcttccggatcttggaaaagaaaa cacctttgagcagtgtgtcagtgaaatggcattggcagggtacactggttccgaagtaggaaataaatatccaaaggacccag aggttctaaaaaaggccctgaaactccggggaatggagatctgcaatcagtggttctcatcatttttgatcacaaagccatttga agaggtggaaaaagagttccgcgctcaactgagtttcttaaaggctgtgggagcaaagatcattggtgcttctgaacagagcc acagtgtacagggtatgctggatactccgatctttggacataagtatgtgatgaatgacgaagagtgggaaaccttctgtacggg aatgaataagttaggaaaaatcgcaaaagaagaatacggcatcagcttaaccttccaccatcatatgggaactgtggtacag gacccggatgaagtggaaagaatgatggcaaacacagatcctgaatatgtgaatctgttatttgatacagggcattttacatact gcggggcagatcctcttgaaatggtgaaaaaatatgtggaccgcatcaaacatgtccatcttaaggatatccgtccggaaatcg tagaaaaagtgaaagcagaaaacttaagcttcttagatggtgtgcgtatgggggcgtttacggtaccgggtgacggatgtattg attttgatccgatctttaaggtgcttgaagattcaggatatgaaggatatatgctggtagaggcagaacaggatccggccaaggc aaatccgcttgaatatgccatgaaggcaagaaaattcatcgctgagaaaactggattataa |
| Amino acid sequence (SEQ ID NO:6) |
| MFNKEKVKLGIAPIAWTNDDLPDLGKENTFEQCVSEMALAGYTGSEVGNKYPKDPEVLKK ALKLRGMEICNQWFSSFLITKPFEEVEKEFRAQLSFLKAVGAKIIGASEQSHSVQGMLDTPI FGHKYVMNDEEWETFCTGMNKLGKIAKEEYGISLTFHHHMGTVVQDPDEVERMMANTDP EYVNLLFDTGHFTYCGADPLEMVKKYVDRIKHVHLKDIRPEIVEKVKAENLSFLDGVRMGA FTVPGDGCIDFDPIFKVLEDSGYEGYMLVEAEQDPAKANPLEYAMKARKFIAEKTGL |

| ***Epi-inositol hydrolase* - *AR1Y2_1105*** |
|---|
| Nucleotide sequence (SEQ ID NO:7) |
| atgagtacaaccagaatgacagtagcacaggctcttgtaaaattccttgacaatcagtatgtggaatttgacggggaagaaac aaaatttgtagaaggtttctttactatcttcggacacgggatcgccgttggtctcggagaggcattggacagcaatcctgggagct taaaggttcttcagggaagaaatgagcagggaatgtgccacgtagcgacagcttacgcaaaacaaaatagaagaaggaa gatcattccttgtgcatcttctgtaggacccggagcggcaaatatggtgacagcagcggctacagctacagtgaacaatattcc |
| gcttttggtagtgccttctgacacatttgccacaagacagccggacccggtgctccagcagttagaaaagagtgacagtctctct acaacaacaaacgacgcattcaaaccggtttgtaaatattgggataggatcatccgcccggaacagctgatgacagccatgg tcaacgctatgcgtgttctcacagatccgtcagagacaggcgctgtgtgcattgcactctcccaggatgtagaaggagaatctta tgatttccctgactatctgtttgaaaaacgtgtacataggatcacaagaccttgcgcggtaaaagaagagatcgaggaccttgta aaagtcattaaagaagcgaaaaatccgttagtgatcgtgggcggaggtgtgaagtattctgaggcaggagaagctgtagaaa agttctgcgaagactttaacatcccgttcggagagagccaggcaggaaaaagtgcctgcaagtcaagccacccttactgctta ggaggaatcggcgtcacaggaaccctggctgccaataccatcgctgatgaagctgatgtggtcattgcagtgggaaccagac tttcagactttacaacaggatctaaaggacagttcaaaaatcctaatgtaaaaatggtgacgatcaacaacaataaatatcatg catataaaatggatgcagtaaaagctgtcggagacgcgaaagtgacagtggaagctctttctgctagactgaaagaggaag gatacaagtctgcttacacaacagagatccaggacgcaaaggcagcctgggataaagaaatggaccgccttgcgaatattg agtatacaggcgatgactttgagccggagatcaaagcgagagatccgcgtacgattcctgaattcgtaaaacttacaggggg acttatcactcagactgcagcattgtgcaaaatccgtgaggtcattgatgaagacgctaatattgtgacagcaggggggagtctt ccaagctgtatgcagagaatctggaccacagacaaacgaggcggatatcacgcagaatacggatattcctgcatgggatatg aagtggcgggagcgctcggcgtaaagatggcagagcctgagcatgaatcctacgtggttgtgggggatgccagctttcagat gctccacagtgagattatgacagcgatgcaggaaaaacaaaagatcaacatccttgtatttgacaactgcggatttggctgtat caacaaccttcagatgaatcatggagtgggaagtcttgcaacagagttcagataccgcgatgaaaacaatgagatcaagggt gatctgatcccggttgactatgcaaaagtcggagaaggatacggactcaagacgtacagtgtaagaacgatcaaagagctg gaggaagcattgatcgatgctaagaaacaggacgttgccacattgatcgacttgaaggtcattccgaaaacaatgacagacg gttacaaatcatggtggaacgtaggtattgcgacaacttctgtgaaagaaagcgtgcagaaagcatgtgaaggagtattggaa ggccgcaaaaacgcaagacagtattag |
| Amino acid sequence (SEQ ID NO:8) |
| MSTTRMTVAQALVKFLDNQYVEFDGEETKFVEGFFTIFGHGIAVGLGEALDSNPGSLKVLQ GRNEQGMCHVATAYAKQNRRRKIIPCASSVGPGAANMVTAAATATVNNIPLLVVPSDTFA TRQPDPVLQQLEKSDSLSTTTNDAFKPVCKYWDRIIRPEQLMTAMVNAMRVLTDPSETGA VCIALSQDVEGESYDFPDYLFEKRVHRITRPCAVKEEIEDLVKVIKEAKNPLVIVGGGVKYS EAGEAVEKFCEDFNIPFGESQAGKSACKSSHPYCLGGIGVTGTLAANTIADEADVVIAVGT RLSDFTTGSKGQFKNPNVKMVTINNNKYHAYKMDAVKAVGDAKVTVEALSARLKEEGYKS AYTTEIQDAKAAWDKEMDRLANIEYTGDDFEPEIKARDPRTIPEFVKLTGGLITQTAALCKIR EVIDEDANIVTAGGSLPSCMQRIWTTDKRGGYHAEYGYSCMGYEVAGALGVKMAEPEHE SYVVVGDASFQMLHSEIMTAMQEKQKINILVFDNCGFGCINNLQMNHGVGSLATEFRYRD ENNEIKGDLIPVDYAKVGEGYGLKTYSVRTIKELEEALIDAKKQDVATLIDLKVIPKTMTDGY KSWWNVGIATTSVKESVQKACEGVLEGRKNARQY |

| ***5-deoxy-glucuronate isomerase* - *AR1Y2_1106*** |
|---|
| Nucleotide sequence (SEQ ID NO:9) |
| atgggtaaagtattcggctatcctgagtttgatgtaaacggggaaaaggttttaacaacatatgacaatgattataaagaaatgct gatggacatccgcgtcttccgcttaaaagcgggagatgtgaagaccttctgcaaagaaaaagaggagaccgcggttcttttact |
| aaaaggggatgtcgtattctcatttgatgaaacttccaaagaagtcacaagaaaagatgtatttacagaaggaccttgggcagt acatatctgcacaggagccaaagtcatagtggaagcaaaggcagactctgaaatcttggtacagtccacaatgaatgaccag acatttgagagtaaattgtatgcgccggaagatgctccttggggatattcctgtgtaggaaagttcggaaatgtggctaaacgcc gtgtcaataccatctttgaccatgacattgcaccatactccaacatggtattaggtgaagtcttaaatgaccgcggaaactggtca ggatatcttcctcacagacatccgcagccggaaacttattacttcctgtttgaccatccggaaggatttggggcaagctttgtggg agaccaggtatttaagagtacagacggaagcttctcagcgattccagggggagaacttcatcctcaggcagtggctccgggat accaaatgtatacttgctggatgatccgtcatctggaagggaacccatggcttcagacagacagaaatgaagacgaacgcta cgtttggcttcatgacgcagaattctaa |
| Amino acid sequence (SEQ ID NO:10) |
| MGKVFGYPEFDVNGEKVLTTYDNDYKEMLMDIRVFRLKAGDVKTFCKEKEETAVLLLKGD VVFSFDETSKEVTRKDVFTEGPWAVHICTGAKVIVEAKADSEILVQSTMNDQTFESKLYAP EDAPWGYSCVGKFGNVAKRRVNTIFDHDIAPYSNMVLGEVLNDRGNWSGYLPHRHPQP ETYYFLFDHPEGFGASFVGDQVFKSTDGSFSAIPGGELHPQAVAPGYQMYTCWMIRHLE GNPWLQTDRNEDERYVWLHDAEF |

| ***5-keto-2-deoxygluconokinase* - *AR1Y2_1104*** |
|---|
| Nucleotide sequence (SEQ ID NO:11) |
| atgaagtatgttgaatttgaccagacaagaccaatggatttagtactcatcggaagggtagccatcgattttaacccggtagatta tttccacccgttatctgagtgcactaccttcaagaagtatgtgggaggatctccggcaaacatcgcagtgggcatcacaaggca cggaaagaaagccggattcatcggcaaagtttctgatgaccagttcggagagtatgtagtagacttcttcaataaagagggaat cgatacatcccatattacaaaatgtacgaacggagaaaagcttggacttacctttacagaaattctttccagagaagaaagca gcatcttaatgtaccgcaactgcatcgcagacttacagttatctgtggatgacattgacgaagaatacataaagaacacaaaa gcaatcctggtttccggaacagcattggcacagagcccatccagggaagcagtgttaaaagcagtgatgcttgccaagaaaa atgatacaaagatcatctttgacattgactatagagaatacaactggaaaaattccgatgagatttcaatctactattctttagtagc cagagaagcagatatcatcatgggatctagagaagaatttgacctgacagaaaaactgataaaaccgggcagaacagaca aagaatctgcacaggcatggaacactgaaaatgcaaagatcgttgtcatcaaacatggcaaaaaaggatctacagcatata ccaatgacggaaatgactattcaatcaaaccgttcccggttgaggcgttaaaaggctttgggggcggagacggatatgcatcc ggcttcttatatggaatatttgaaggatgggatatgatcgagtgtctggaattcggaagtgcggaagcggccatgatggtgagaa gccattcttgttctgaagaacttccaaaccctcaggaagtcaaagactttatccgcgagacaaaagacaaatatggcgaaatg attgcgagagtatag |
| Amino acid sequence (SEQ ID NO:12) |
| MKYVEFDQTRPMDLVLIGRVAIDFNPVDYFHPLSECTTFKKYVGGSPANIAVGITRHGKKA GFIGKVSDDQFGEYVVDFFNKEGIDTSHITKCTNGEKLGLTFTEILSREESSILMYRNCIADL QLSVDDIDEEYIKNTKAILVSGTALAQSPSREAVLKAVMLAKKNDTKIIFDIDYREYNWKNSD EISIYYSLVAREADIIMGSREEFDLTEKLIKPGRTDKESAQAWNTENAKIVVIKHGKKGSTAY TNDGNDYSIKPFPVEALKGFGGGDGYASGFLYGIFEGWDMIECLEFGSAEAAMMVRSHS CSEELPNPQEVKDFIRETKDKYGEMIARV |

| ***5-keto-2-deoxy-D-gluconate-6 phosphate aldolase* - *AR1Y2_1054*** |
|---|
| Nucleotide sequence (SEQ ID NO:13) |
| atggcattagtcaaaatgaaacagttattagagcaggcaaaaaaggaaaacagaggatgcggtgcattcagtgtgggcaat atggaaatggtcaaaggggcgattcaggcagcggaggagatgaatacaccgatcatcctgcagatcgctgaagtgcggctc gcccattcaccgcttcatcttatggggccgatgatggtgcaggcggcaaaagaggcaaaggtggatgtggcagttcatctgga ccacggacttaacattgagactgtaaaaaaagcgctggaactgggatttacctctgttatgctggatgcatccagagattcgtttg aagaaaatatcagaaaaacaaaagaagttgtcgctctggcagcacagtacggagcaaccgtggaagcggaattagggctg gtaggcggaagcgaagacggaaagagtgaccatggaatccagtgcaccaagcctcaggacgccaaggtcttctgtgagca gaccggagtggatgccctggccgttgccattggaaatgcccacggggactatccggtagctccgactttggaatttggtgtattg gaagacattcacaatattactgccaaaccattggtgcttcacggcggaagcggtattacagatgaggatttcagaaaagccatc tctcttggaatctgcaaagtgaacattgcaactgcaagctttaacagcctgacaaaagaagtggaaaactatctggagacaga aggaaaacataattacttcgatatgaacgaagctatggtaaagggtgtctatgacaacgtaaaacgccatatccagatctttaa ctgcagataa |
| Amino acid sequence (SEQ ID NO:14) |
| MALVKMKQLLEQAKKENRGCGAFSVGNMEMVKGAIQAAEEMNTPIILQIAEVRLAHSPLHL MGPMMVQAAKEAKVDVAVHLDHGLNIETVKKALELGFTSVMLDASRDSFEENIRKTKEVV ALAAQYGATVEAELGLVGGSEDGKSDHGIQCTKPQDAKVFCEQTGVDALAVAIGNAHGD YPVAPTLEFGVLEDIHNITAKPLVLHGGSGITDEDFRKAISLGICKVNIATASFNSLTKEVENY LETEGKHNYFDMNEAMVKGVYDNVKRHIQIFNCR |

| ***Glyceraldehyde-3-phosphate keto-isomerase* - *AR1 Y2_1336*** |
|---|
| Nucleotide sequence (SEQ ID NO:15) |
| atgcgtaaaacaatcgtagcaggaaactggaaaatgaacaaaacacctagcgagacagtagctttagttaatgaattaaagc cattggtagcaagcgaggtttcagatgttgtcttctgtgtgccggccatctgtattccggcagcactggaagctgcaaaaggaagt aacatcaacatcggcgctgagaacatgtattttgaagagagcggcgcttacacaggcgagatcgctccgaatatgttaacaga cttaggtgtgaaatatgttatcatcggacattctgagagaagagaatattttgcagaaacagatgagacagtgaataaaaaagt cttaaaagcttttgaacatggaattactccaatcgtttgctgcggtgagacacttgctcagagagagcagggtgtgaccatcgatt tcgtacgccagcagatcaagatcgcattccagggtgtgactgctgaccaggcaaagacagctgtcattgcatacgaaccgatc tgggctatcggaacaggaaaagttgcgacgacagaacaggctcaggaagtatgtaaagcgatccgtgagtgcatcgctgaa gtatacgatcaggcaacagcggacgctattcgtatccagtacggcggttctgtatccgctgcaagtgcaccagaattatttgcac aggaagacatcgatggaggattagtcggtggagctgctttaaaaccagactttggtaagatcgtgaattacaaataa |
| Amino acid sequence (SEQ ID NO:16) |
| MRKTIVAGNWKMNKTPSETVALVNELKPLVASEVSDVVFCVPAICIPAALEAAKGSNINIGA ENMYFEESGAYTGEIAPNMLTDLGVKYVIIGHSERREYFAETDETVNKKVLKAFEHGITPIV CCGETLAQREQGVTIDFVRQQIKIAFQGVTADQAKTAVIAYEPIWAIGTGKVATTEQAQEV |
| CKAIRECIAEVYDQATADAIRIQYGGSVSAASAPELFAQEDIDGGLVGGAALKPDFGKIVNY K |

| ***Glyceraldehyde-3-phosphate dehydrogenase* - *AR1Y2_1334*** |
|---|
| Nucleotide sequence (SEQ ID NO:17) |
| atggcagtaaaagtagcgattaatggatttggacgtattggacgtcttgcattcagacagatgttcggagcagaaggttatgaag ttgtagcaatcaacgatttaacaaatcctaagatgttagcacatttattaaagtatgattctgcacagggaagatatgcattaggcg acaaggtagaagcaaaagaatcttccatcgtagttgacggaaaagaaatcgaaatctacgcagaagcagacgcaacaaa acttccttggggagaattagatgttgatgttgtattagaatgtacaggattctacacatctaaagacaaagcatccgcacacgttc aggcaggagctaagaaagttgttatttctgcgccagctggaaatgatcttccaactatcgtatataatgtaaaccacgatacattg acaaaagaagacacagtcatctcagcagcatcttgtacaacaaactgcttagcacctatggctgatgcactgaacaagtatgc accaatccagtctggtatcatgttaacagtacatgcttacacaggagatcagatgactcttgacggacctcagagaaaaggtga cttaagaagagcacgtgcagctgcagtcaacatcgtacctaactctacaggtgctgcaaaagctatcggattagttatcccaga attaaacggaaaattaatcggatctgctcagcgtgtaccggttcctacaggatcaacaacacttttaacagcagttgttaaaggt gacaatgtaactgttgaaggaatcaacgcagctatgaaagcagcagcaacagagtctttcggatacacagaagaagaatta gtatcttctgacatcatcggaatcaaatatggttctttattcgacgcaactcagacaatggttactgaagttggtgacggattatatg aagtacaggttgtttcttggtatgacaacgaaaactcatacacaagccagatggttagaacaatcaaatactttgcagaattagc ataa |
| Amino acid sequence (SEQ ID NO:18) |
| MAVKVAINGFGRIGRLAFRQMFGAEGYEVVAINDLTNPKMLAHLLKYDSAQGRYALGDKV EAKESSIVVDGKEIEIYAEADATKLPWGELDVDVVLECTGFYTSKDKASAHVQAGAKKVVIS APAGNDLPTIVYNVNHDTLTKEDTVISAASCTTNCLAPMADALNKYAPIQSGIMLTVHAYTG DQMTLDGPQRKGDLRRARAAAVNIVPNSTGAAKAIGLVIPELNGKLIGSAQRVPVPTGSTT LLTAVVKGDNVTVEGINAAMKAAATESFGYTEEELVSSDIIGIKYGSLFDATQTMVTEVGDG LYEVQVVSWYDNENSYTSQMVRTIKYFAELA |

| ***Phosphoglycerate kinase* - *AR1Y2_1335*** |
|---|
| Nucleotide sequence (SEQ ID NO:19) |
| atgttaaacaagaaatcagtagatgatatcaacgtaaaaggaaaaaaagtcttagtgcgctgtgacttcaacgttccgcttcag gatggaaagatcacagatgagaaccgtcttgtggcagcacttcctacactaaagaagttaattgctgacggcggaaagctgat cctctgctcacacttaggaaaaccaaaaggggaaccgaaaccggagttatctcttgcgccagtagcagaaagattaagcga gttattaggacaggaagtaaagtttgctgcagatgacaacgtggtaggaccgaatgcaaaagctgcggtagaaggaatgca ggacggggatgtcattttacttcagaatacacgctacagagcagaagagacaaagaatggagaagagttcagcaaagaact tgcaagtctctgcgatgtttttgttaatgatgcttttggtaccgctcatagagctcactgctctaacgtgggagttacaaagttcgtgg acactgcagttgtaggatatttaatgcagaaagagattgatttcttaggaaatgcggtcaacaatccggaacgtcctatggtcgct atcttgggaggagcaaaggtttcctctaagatctctgtgatcaataacctgctagacaaagtcgatactttgatcatcggcggag gtatggcatatactttcatgaaagcattgggagaagaagttggagattcccttctggaagctgactacttagactacgcaaaaga |
| aatgatggacaaggcaaaagaaaaaggtgtgaacttactgattcctgttgacactgtagttgctgatgacttctcaaatgacgcc aacatcaaaactgttgagagaggcggaatcgaagcaggatggcagggacttgacatcggaccaaagacaagagaactgtt ttcaaatgcactgaaagatgccaagactgtagtatggaacggaccgatgggatgctttgagatgccgaactttgcagcaggaa ccgttgcagttgcagaagcactggcagagatcgacgctacaactatcattggcggaggagattccgcagcggcagttaacca gctcggattcggagataagatgactcatatctctacaggtggtggagcatcactggaattcctggaaggaaaagaattaccag gcgttgcagcggcaaacgataaataa |
| Amino acid sequence (SEQ ID NO:20) |
| MLNKKSVDDINVKGKKVLVRCDFNVPLQDGKITDENRLVAALPTLKKLIADGGKLILCSHLG KPKGEPKPELSLAPVAERLSELLGQEVKFAADDNVVGPNAKAAVEGMQDGDVILLQNTRY RAEETKNGEEFSKELASLCDVFVNDAFGTAHRAHCSNVGVTKFVDTAVVGYLMQKEIDFL GNAVNNPERPMVAILGGAKVSSKISVINNLLDKVDTLIIGGGMAYTFMKALGEEVGDSLLEA DYLDYAKEMMDKAKEKGVNLLIPVDTVVADDFSNDANIKTVERGGIEAGWQGLDIGPKTR ELFSNALKDAKTVVWNGPMGCFEMPNFAAGTVAVAEALAEIDATTIIGGGDSAAAVNQLG FGDKMTHISTGGGASLEFLEGKELPGVAAANDK |

| ***Phosphoglycerate mutase* - *AR1Y2_0899*** |
|---|
| Nucleotide sequence (SEQ ID NO:21) |
| ttgaagttatatatgatgaggcacggagaaactccatggaataaagtgcggaggattcaggggcagtcagacgtggatctga gtgaagcgggcagggaagcagccaggctcaccaggagagcactgaaagatttccattttgatgcggcctacacgagcccg ctgagcagagcgagagaaacaggggagatcatcctggagggcagagggatccctctgctggaggatgaacggcttaagg aggccaacttcggaccttatgaaggggcacacatcaatgaattatatgaaagagaagaaccgattctggatttctttgaccggc cggaacagatggcctatattgaaggagcggagactccggctcaggtagtagaaagatccaggatatttttagaggaatttatac ttccgatggaaaaagaagcagaaagtgtgatcttatttacccacggagctttcatccacgggatgctgactaatatgtatcaaag agagatcagggacttttggaaaggaccgaagcttaagaattgttctatcagtacggctgaagtcagggacggtcagttccgcat tttgagtgaggctgaggtcttttactga |
| Amino acid sequence (SEQ ID NO:22) |
| MKLYMMRHGETPWNKVRRIQGQSDVDLSEAGREAARLTRRALKDFHFDAAYTSPLSRAR ETGEIILEGRGIPLLEDERLKEANFGPYEGAHINELYEREEPILDFFDRPEQMAYIEGAETPA QVVERSRIFLEEFILPMEKEAESVILFTHGAFIHGMLTNMYQREIRDFWKGPKLKNCSISTA EVRDGQFRILSEAEVFY |

| ***Enolase*** - ***AR1Y2_0982*** |
|---|
| Nucleotide sequence (SEQ ID NO:23) |
| atggattatttagagattgaaaaggtcataggaagggaaattattgattccaggggaaatcctacagtggaagcagaagttctttt agccggcgggacaatcggacggggcacagcacccagcggagcttcaactggagaatttgaggcgttggaattaagagacg gagataaggagcgttttggaggaaaaggtgtgtccaaggcagttcataatattaatacgatcattaacgatactttaaaagggat ggatgcatccgatacttacgcggtggacagagcgatgatcagcgtagacggaacaaaggataaatcaaagcttggggcca |
| atgcgatccttgcggtttcgattgcatgcgcaaaggcggcatcggcaggcctggatctgccattataccgttttctgggcggtgtta atgccaaccgtcttccggtgccgatgatgaacattttaaacggaggtgcccatgcggccaacaccgtcgatgtacaggagttta tgatcatgcctgtgggagcagaaaatttcagggaagcactccggtggtgtacggaagtattccacgccttggcagctttgctgaa atccaaagggctggcaacctccgtgggtgatgaaggcgggtttgcgcctgaccttgcaagtgatgaggaagccatcgaatata ttttggaagctgttcaggcagcgggatatgagccgggcagtgattttgttctggctatggatgctgcttccagcgagtggaaaggc gcgaacaaaggtgaatatattcttcctaagagtggcaagaaatttacctcagaagaactggtggaccactggaaatcactttgt gagaaatatccgatcagctccattgaggatggattggatgaggaagactgggacggctggaaatacatgacccaggagcttg gagagaaaatccagctggtgggagatgacttatttgtaacgaatacggaacgccttggaaaaggtatcaagatgggatgcgg aaattctatcttgatcaagctgaaccagatcggttctgtgtcggagacattggaagctattaagatggcccataaagcgggttac acagcggtcacatcccaccgttcaggggagacagaagacacgaccatcgcagatctcgctgttgcacttaataccggacag atcaaaaccggtgcaccgagcagaagtgagagggtggcaaaatataatcagcttctgaggattgaagaggaactggggga aagtgccgtatacccgggagtcggaacattttag |
| Amino acid sequence (SEQ ID NO:24) |
| MDYLEIEKVIGREIIDSRGNPTVEAEVLLAGGTIGRGTAPSGASTGEFEALELRDGDKERFG GKGVSKAVHNINTIINDTLKGMDASDTYAVDRAMISVDGTKDKSKLGANAILAVSIACAKAA SAGLDLPLYRFLGGVNANRLPVPMMNILNGGAHAANTVDVQEFMIMPVGAENFREALRW CTEVFHALAALLKSKGLATSVGDEGGFAPDLASDEEAIEYILEAVQAAGYEPGSDFVLAMD AASSEWKGANKGEYILPKSGKKFTSEELVDHWKSLCEKYPISSIEDGLDEEDWDGWKYM TQELGEKIQLVGDDLFVTNTERLGKGIKMGCGNSILIKLNQIGSVSETLEAIKMAHKAGYTA VTSHRSGETEDTTIADLAVALNTGQIKTGAPSRSERVAKYNQLLRIEEELGESAVYPGVGT F |

| ***Pyruvate-flavodoxin oxidoreductase* - *AR1Y2_3042*** |
|---|
| Nucleotide sequence (SEQ ID NO:25) |
| atggcaagaaaaatgaaaaccatggatggtaacacagctgcagcacacgtgtcttacgcttttactgagacagctgctatctac ccaattacaccatcctcaccaatggcagaatcaacagaccagtgggcaacacagggaaagaaaaatattttcggacatcct gtaaaattagttgaaatgcagtcagagggcggagcagcaggaactgttcacggatctcttgcagcaggcgcattgacaacta catacactgcatctcagggattattactgatgattcctaatatgtacaagatcgcaggtgaattaatgccttgtgtatttgatgtatctg cacgtgcaatcgcatctcatgcactgaatatctttggtgatcactctgacgtttacgcttgtcgccagacaggttttgcaatgctttgc agctccagcgttcaggaagttatggacttaggcgctgttgctcatttatctacaatcaagggaagcgttccagttctccatttcttcg atggattcagaacatctcatgagatccagaagattgaaatgtgggacaacgaagatcttgcagatatggtagattacgatgctct tgaaaagttcagagcaaatgctttgaatccagaacatccggttctccgcggatctgctcagaatccagatatcttcttccaggca agagaagcatgcaacaaatactacgatgcattcccggcaatcgtcgaagattacatgaaccaggttaatgcaaagatcggta cagattataaattattcaactactacggagctgctgatgctgaatctgttatcattgcaatgggatctgtatgtgagactatcgatga gaccatcgattacttaacagcaaaaggcgaaaaagtcggtgttgttaaagtaagactttacagaccattcgtagctgaaaaatt actcgaagttatcccagagactgttaaaacaatcaacgttttagacagaacaaaagaaccgggatctcttggagagccattat acttagacgttgtttctgcattaagaggaacaaaattcgagcaggtaacaatcaacggaggacgttacggattaggatccaaa |
| gatacaactcctgctgatatcatctctgtattcaataacaaagataagaaagaattcacagttgcgatcaacgatgatgtgacaa acttatctcttgaaagaggagaagctgtttcttctacaccagaaagcacagtaagctgtaagttctggggtcttggagcagatggt accgttggtgctaacaagaactccatcaagatcatcggtgaccacactgacaaatatgctcaggcatactttgattatgattcca agaaatccggtggtgttacaatttcccacttacgtttcggcgatgacccgatcaaagcaacttacctgatcaacaaagctgatttt gttgcatgtcactgtgctccgtacatgacaaaatacaacatggttcaggacttaaaagatggcggaacattcttgttaaactgtag ctggccggtagaagaaatcggagatcatcttccaggacaggtaaaagcttacatggcaaaacacaacatcaagttctatgtg atcgatggtatcaagatcggtaaagagattggacttggcggacgtatcaatactgtacttcagtctgcattcttcaaattatctgga attattccggaagcagacgcgatccagtatatgaaagacgctgctactgcttcttacagcaagaagggtgatcacatcgttaag atgaatcatgacgcgatcgaagctggtgctaagagtgttgtagaagttccggttcctgctgaatgggctgactgcggagaagaa agcttattacatccagtatccggagatagaaaagatcttgttgattatgttaacaacattcagaacgctgtcaatggacagattggt aacagccttaaggtaagcgacttagttccttacgctgacggtcatatgcctcagggatctgctgcatacgaaaaacgtggagttg ctgtagacgttcctacatggaatccggacaactgtatccagtgtaacttctgtgcatatgtatgtcctcatgcagttatccgtcctgtt gccatgacagaagcagaacttgctgcagcaccggaagctacaaaatctcttccgatgacgggaatgccagattacaaatttgt aatgacaactactgttcttgactgtacaggatgtggttcttgtgcaaatgtctgcccaggtaagaaaggcgagaaagctcttaca atggcaccactcgacagcatgagagatcagcaggaagtttacaactatggacagtctttacctataaaagaagatatctttgac aagtttaaaccagaatctgttaagggaagccagttcaagaaacctctccttgagttctcaggagcttgtgcaggatgtggagag actccatatgcgaaactgatcactcagttattcggagacagaatgtacattgccaatgcgacaggatgttcttctatctggggtggt tcttcacctgcaacaccttacacagtaaacagcgaaggacagggaccggcttggtctaactcactgttcgaagacaatgctga gtttggtttaggtatgttcacaggacagaatactttaagagagcagacaaaagaaaaggttgcagcattagcagaatctacag ataatgcagatgtaaaagcagcagcggctgaatacttagatacttacacagacggtaagaaaaacggagcagctactaaga aattagttgcagcactggaagcttgcggatgtgaagaaggaaaagaaatccttaagatgaaagattacttatctaagaaatctc agtggatcttcggtggtgacggatgggcttacgacatcggctttggcggtgttgaccatgtacttgcaagtggtgaagatgttaata tctttgtattcgatacagaggtttactctaacaccggcggacaggcatccaaatctacaccggcaggtgcgatcgctcagttcgc agcagcaggtaaggctgtgaagaaaaaagaccttgcagcaatcgcaatgagctacggatatgtatatgttgctcagatctctat gggtgctgattacaatcagacagtcaaagcgatcgcagaggcagaagcttatccaggaccatcactgatcatcggttatgctc catgtatcaaccacggtatcaagggtggaatgagcattgctcagtctgaagaaaagaaagcagttacatcaggatactggaa cttattccgtttcaaccctgaattagctgaagaaggaaagaatccattctcattagattctaaagagcctacaacagagtacaatg acttcatcatgggtgaggttcgttacaacgctcttgctcgccagaatcctgagagagctaaggagctgtttgagcaggcagaaa agaatgccaaagaaaaatatgctcgtttgaaagaattagcagataaataa |
| Amino acid sequence (SEQ ID NO:26) |
| MARKMKTMDGNTAAAHVSYAFTETAAIYPITPSSPMAESTDQWATQGKKNIFGHPVKLVE MQSEGGAAGTVHGSLAAGALTTTYTASQGLLLMIPNMYKIAGELMPCVFDVSARAIASHAL NIFGDHSDVYACRQTGFAMLCSSSVQEVMDLGAVAHLSTIKGSVPVLHFFDGFRTSHEIQ KIEMWDNEDLADMVDYDALEKFRANALNPEHPVLRGSAQNPDIFFQAREACNKYYDAFPA IVEDYMNQVNAKIGTDYKLFNYYGAADAESVIIAMGSVCETIDETIDYLTAKGEKVGVVKVR LYRPFVAEKLLEVIPETVKTINVLDRTKEPGSLGEPLYLDVVSALRGTKFEQVTINGGRYGL GSKDTTPADIISVFNNKDKKEFTVAINDDVTNLSLERGEAVSSTPESTVSCKFWGLGADGT |
| VGANKNSIKIIGDHTDKYAQAYFDYDSKKSGGVTISHLRFGDDPIKATYLINKADFVACHCA PYMTKYNMVQDLKDGGTFLLNCSWPVEEIGDHLPGQVKAYMAKHNIKFYVIDGIKIGKEIG LGGRINTVLQSAFFKLSGIIPEADAIQYMKDAATASYSKKGDHIVKMNHDAIEAGAKSVVEV PVPAEWADCGEESLLHPVSGDRKDLVDYVNNIQNAVNGQIGNSLKVSDLVPYADGHMPQ GSAAYEKRGVAVDVPTWNPDNCIQCNFCAYVCPHAVIRPVAMTEAELAAAPEATKSLPMT GMPDYKFVMTTTVLDCTGCGSCANVCPGKKGEKALTMAPLDSMRDQQEVYNYGQSLPIK EDIFDKFKPESVKGSQFKKPLLEFSGACAGCGETPYAKLITQLFGDRMYIANATGCSSIWG GSSPATPYTVNSEGQGPAWSNSLFEDNAEFGLGMFTGQNTLREQTKEKVAALAESTDNA DVKAAAAEYLDTYTDGKKNGAATKKLVAALEACGCEEGKEILKMKDYLSKKSQWIFGGDG WAYDIGFGGVDHVLASGEDVNIFVFDTEVYSNTGGQASKSTPAGAIAQFAAAGKAVKKKD LAAIAMSYGYVYVAQISMGADYNQTVKAIAEAEAYPGPSLIIGYAPCINHGIKGGMSIAQSE EKKAVTSGYWNLFRFNPELAEEGKNPFSLDSKEPTTEYNDFIMGEVRYNALARQNPERAK ELFEQAEKNAKEKYARLKELADK |

| ***3-oxoacid CoA transferase* - *AR1Y2_1115*** |
|---|
| Nucleotide sequence (SEQ ID NO:27) |
| atgggaaaagttaaaattatcacagcagatcaggctgctgccctggtggaggatcatacaacgatcacaaccagcggatttgt tgccagcggaatgccggaagctctcacaaaagctttggaaaaaagattcaaagaaaccggatcccccaaaaacctgacttt gttttatgctgccgcccagggaaaccgtgacggaagcggcgcggatcattttgcccatgaaggtatgacaaaacgtgtcatag gcggacattggaacatggttcctgcactgggacagatggttctggacaataaaatcgaaggctataatctgcctcagggaaca ctggcacagttataccgtgccatcgcaggtcacaaacccggcgttatcagtcatgtaggattaaatacatttgctgacccacgca ttgagggcggtaaattaaatgacattacaaccgaagatatcgtcgatgtagttgaaatcctgggagaagagaaattattgtaca aatcgttcccgatcaacatcggatttatccgcggtacatatgcagatgaacacggcaacgtaactctgtctcacgaatgtgccac cacagaggtcacaaccatggctcaggccgtaaagaacagcggcggaaaggtcgttgtacaggtagaaaaggtcgtttcag acggcaccttagatccaaaattagtcaagatccctggaatttatgtggatgccgttgtggaagtagaagacatgaaggatcacg aacaatgcgtgggctgtgattatgatggttccatgaccggagacttccgggttccgttaagcagtcttgagtacccgcctctttccg ccaagaagatcattggacgcagagcagccatggaactgacagaaaacacggttgtaaatctaggcattggtattccagagta catctccatggtagccaacgaagaaggcattggagattacatgactctgactgtagaggccggaccaatcggcggtgtacctc agggaggagctaaattcggaggcgctgtcaatgctgaatgtatcctagatcagccgtaccagtttgatttctatgacggcggcg gagttgactatgcattcttaggactcgcacaggcagataaagacggtaatatcaacgtaagcaagttcggtccaaggattgcg ggctgcggcggtttcgtcaatatcacccagaacgcaaaacgctgctacttctgcggaacttttactgcaggaggtctgaagaca tccgtaaaagacgggaaacttatcattgaccaggaaggaaaatcaaacaaattcctggataccgtagaacagatcaccttca gcggtgaatacgcaaataaagtcggccagccggttctctatatcacagaacgtgctgtattcgaactcagaaaggacggcgtc tatctgacagaggttgcccctggtatcgatatccagacacagatcatcgatcatatgggatttgctccaaagatggaaggaacg ccaaagttcatggatgaaagaatcttcaaagatgagctgatgggcctgaagcacgattaa |
| Amino acid sequence (SEQ ID NO:28) |
| MGKVKIITADQAAALVEDHTTITTSGFVASGMPEALTKALEKRFKETGSPKNLTLFYAAAQG NRDGSGADHFAHEGMTKRVIGGHWNMVPALGQMVLDNKIEGYNLPQGTLAQLYRAIAGH KPGVISHVGLNTFADPRIEGGKLNDITTEDIVDVVEILGEEKLLYKSFPINIGFIRGTYADEHG NVTLSHECATTEVTTMAQAVKNSGGKVVVQVEKVVSDGTLDPKLVKIPGIYVDAVVEVED MKDHEQCVGCDYDGSMTGDFRVPLSSLEYPPLSAKKIIGRRAAMELTENTVVNLGIGIPEY ISMVANEEGIGDYMTLTVEAGPIGGVPQGGAKFGGAVNAECILDQPYQFDFYDGGGVDYA FLGLAQADKDGNINVSKFGPRIAGCGGFVNITQNAKRCYFCGTFTAGGLKTSVKDGKLIID QEGKSNKFLDTVEQITFSGEYANKVGQPVLYITERAVFELRKDGVYLTEVAPGIDIQTQIIDH MGFAPKMEGTPKFMDERIFKDELMGLKHD |

| ***Acyl-CoA dehydrogenase* - *AR1Y2_1117*** |
|---|
| Nucleotide sequence (SEQ ID NO:29) |
| atgaattttgaattaacagagcagcaggcagccattcaggaaacagcaaggaactttgctcagacagaactgcagccaggt gtgctggagagagatgcaaacagtgagtttccggtagatctttataagaagatgggggatatgggcctgatcggtcttccatatc cgaaagaagtcggaggacagggagccgattatctttcttatgcactggcagtggaagagatctcaaaagtagatgcctctgtg ggtatttcctattctgtttccacctctctgtatggcggaagcattatgaactctgatgcttccgttgagaaaaagaatgaatttctagca ccggtactgtccggacagcatttcggatcctttggactcacagagcccaatgccggatcagacgcaggcggatgtgtcactgtg gctgaaaaagatggggacgaatacatattaaatggtatgaagtgttttaacaccaacggacctttggccgattatacagcggtat atgccctgacagaacctgagaagaaagcaaagggtctttcctgctttgtagtaaaaaaaggaactcctggattttctgtaggca aggtggaggataagatgggaatccgctctgcgcaggtctctgagatgatcctggaaaatgtcagagtacctgcagagaatatg gtcgttccatctggggacggatttaagctggccatgaagactttggatggaggacgcatcggcgttgccgcgcaggggcttggt atcgcggagggcgcatttgagatcgcaaaagaatacttaaagagcagagaacagttcggcaaaccattatataaaaaccag tatcttgcatttaagatggcggaacttgaaatggagatcgacgctgcaagatacatgctttacaaagcggctacagataaacac gagggaagatcttactcaatcccggcagcaaaggcaaaatacctttgtacagaggccgctatgcatgtgacaacagaagca gtacagatgttaggtggaaatgggtatatgaagggttaccatgtagaacgtatgatgagagatgcgaagatcactcagatctat gagggaaccaatgaaatccagaagctcgttgtaagcggagctatcttcagatag |
| Amino acid sequence (SEQ ID NO:30) |
| MNFELTEQQAAIQETARNFAQTELQPGVLERDANSEFPVDLYKKMGDMGLIGLPYPKEVG GQGADYLSYALAVEEISKVDASVGISYSVSTSLYGGSIMNSDASVEKKNEFLAPVLSGQHF GSFGLTEPNAGSDAGGCVTVAEKDGDEYILNGMKCFNTNGPLADYTAVYALTEPEKKAK GLSCFVVKKGTPGFSVGKVEDKMGIRSAQVSEMILENVRVPAENMVVPSGDGFKLAMKTL DGGRIGVAAQGLGIAEGAFEIAKEYLKSREQFGKPLYKNQYLAFKMAELEMEIDAARYMLY KAATDKHEGRSYSIPAAKAKYLCTEAAMHVTTEAVQMLGGNGYMKGYHVERMMRDAKIT QIYEGTNEIQKLVVSGAIFR |

| ***Enoyl-CoA hydratase* - *AR1Y2_1116*** |
|---|
| Nucleotide sequence (SEQ ID NO:31) |
| atgaacaatttattaatggaagtagaaaacgaagtcgcagttgtaacaatcaacagaccaaaatcattaaacgctttaaacagt gaaactttagctgagttagaccagtgctttacagagatttcaggacgcaaggacatcagagtggtgatcctcacaggatcagg cgaaaaatcctttgtagctggtgctgatatctctgaaatggtcaatgcaactcctgcagaaggaagacagatgggtcttttggca aaagaagcatttcttaagttagagacaatgcctcaggtaaccatcgctgccgtcaacggttacgctctgggaggcggatgcga gatctccatggcatgtgatatccgtgtagctgcggaaaacgcaaaattcgcacagccagaaacaggacttggaattcttccgg gattcggcggaacacagcgtctctcccgtttagtaggaaaaggacgcgcaaaggaattgatctttacatgtgaccagatcgatg ccgaagaagcttacagaattggtcttgcaaacaaagtagtgcctcaggcggagctgatcgattactgtaagaagatggctgca aagatcatgtccaaaggaagctacgcaatttcccttgcaaaagaagcgatcaacacaggaatggacacagacttaagcagc ggtcttacattagaagctgacttattcggacttgcattctccactgctgacaaaaaagagggtatgacagcattccttgaaaaacg taaagctgatttaaaagatttctag |
| Amino acid sequence (SEQ ID NO:32) |
| MNNLLMEVENEVAVVTINRPKSLNALNSETLAELDQCFTEISGRKDIRVVILTGSGEKSFVA GADISEMVNATPAEGRQMGLLAKEAFLKLETMPQVTIAAVNGYALGGGCEISMACDIRVAA ENAKFAQPETGLGILPGFGGTQRLSRLVGKGRAKELIFTCDQIDAEEAYRIGLANKVVPQA ELIDYCKKMAAKIMSKGSYAISLAKEAINTGMDTDLSSGLTLEADLFGLAFSTADKKEGMTA FLEKRKADLKDF |

| ***Acryloyl-CoA dehydrogenase* - *AR1Y2_1050*** |
|---|
| Nucleotide sequence (SEQ ID NO:33) |
| atggttgaatttaaattgacaaaagaacagctgcttgcacagcagatgttcaaagatttcgcagagacagaaatcaagccaatt gctagggacatggatgaaacagaagaattcagcatggaactgcttgagaaaatgaaaaaattcggtcttatgggaattccata ttccaaagaatacggcggagaaggggcagacgttttaacttatacactctgtatggaagagatgtctaagttagatgcttccaca ggaatcacaatttccgtacatacatcattgtgctgttcctgcatcaatgattttgcttcagaagagcagaaacaaaaatttttacgtc cgttggttgacggttctaaagtgggatgctttggtcttacagagccgggagcaggtaccgatgccagcggtgtgaagacaaatg ccgtattggacggcgatgaatatatcttaaacggacagaaggtgttcactacaaactcaggctttgccgatacatttattatttttgc agttacagacaaaactgcaggtacaagaggtatgtctgcatttatcgtggacaggaatacaccgggattagaagtcagcaag aatatccctcgtatgggtatccgtgctgcgtcaaactgtgaagttgctttagttgatgtgagagtaccgaaggaaaacctgatcgc gggcgagggaaaaggatataagatcgctatgtcagcgctggcaggcggacggatcggaatcggcgctcagtccgtaggaa tcgcacagggtgctttaaacgaggccattgcatatgtaaaggaaagaaaacagttcggaaaaccgatttctaaattccagaat acacagttcctgatcgcagaactccaagtgaagatcgacgctgcgcgccttatggtatggagagcggcaaaatcaaaagac gatcatgaaaactatgctcctctggcagcagcgtgcaaactgtttgcatctgacgtggcagtggaagtgacaagagcagccgt acagcttcttggaggatatggatactccagagaatatccggtagagagaatgtaccgtgatgctaagatcacagagatttacga aggtacatctgaagctatgaagatgatcgtcggcggcggacttttaagataa |
| Amino acid sequence (SEQ ID NO:34) |
| MVEFKLTKEQLLAQQMFKDFAETEIKPIARDMDETEEFSMELLEKMKKFGLMGIPYSKEYG GEGADVLTYTLCMEEMSKLDASTGITISVHTSLCCSCINDFASEEQKQKFLRPLVDGSKVG CFGLTEPGAGTDASGVKTNAVLDGDEYILNGQKVFTTNSGFADTFIIFAVTDKTAGTRGMS |
| AFIVDRNTPGLEVSKNIPRMGIRAASNCEVALVDVRVPKENLIAGEGKGYKIAMSALAGGRI GIGAQSVGIAQGALNEAIAYVKERKQFGKPISKFQNTQFLIAELQVKIDAARLMVWRAAKSK DDHENYAPLAAACKLFASDVAVEVTRAAVQLLGGYGYSREYPVERMYRDAKITEIYEGTS EAMKMIVGGGLLR |

| ***Acryloyl-CoA dehydrogenase*/*Etf* - *Electron transfer flavoprotein, beta subunit - AR1Y2_1051*** |
|---|
| Nucleotide sequence (SEQ ID NO:35) |
| atgaaaatcgttgtttgtgcaaaacaagtaccagatacaactgaagtaaaattagatccaaagaccaacactctgatcaggga tggtgttccgagtatcatcaacccagatgacaaagccggaatcgaagctgccctgcagttaaaggaaaaatgcccgggaag cactgtgactgttgtttccatgggaccgcctcaggcagatgtggcattgagagaagcacttgctatgggatgtgacgatgcagttc tggtttctgacagagcattcggaggagcagatacatgggcaacatcttccactatcgcggcagcgatcaaaaaattggattacg atgtcatcatcacaggacgccaggcgattgacggagataccgctcaggtaggccctcagatcgcagaacatcttggaattcct caggtgagctatgtcgaagccctgaatgtggaaggggaagacactttgatcgtccagaggcagtttgaagaccgccatcaga tcattgaagtaaagactccttgcctgatcactgctttggctgaattggcaacacctagatatatgacagtccacggtgcatttgacg catacagagaaaaagaagtcagcgtctggggacttgcagacttagaagacactgtagaccaggcaaatatcgggttaaaag gatctcctacaagagtaaagaaatcctttacaaaacaggccaaaggtgcaggaactattttaaaagatttatcagcagacgaa gctgttgcatccattgttgataaacttcaggaaaagcacatcatttaa |
| Amino acid sequence (SEQ ID NO:36) |
| MKIVVCAKQVPDTTEVKLDPKTNTLIRDGVPSIINPDDKAGIEAALQLKEKCPGSTVTVVSM GPPQADVALREALAMGCDDAVLVSDRAFGGADTWATSSTIAAAIKKLDYDVIITGRQAIDG DTAQVGPQIAEHLGIPQVSYVEALNVEGEDTLIVQRQFEDRHQIIEVKTPCLITALAELATPR YMTVHGAFDAYREKEVSVWGLADLEDTVDQANIGLKGSPTRVKKSFTKQAKGAGTILKDL SADEAVASIVDKLQEKHII |

| ***Acryloyl-CoA dehydrogenase*/*Etf* - *Electron transfer flavoprotein, alpha subunit - AR1Y2_1052*** |
|---|
| Nucleotide sequence (SEQ ID NO:37) |
| atgagcagagacgtatatgtattcgctgagcagagagacggcgaacttcagaaagtcggaatcgaattaattggtaaagcaa gagaattagcagatgatcttggacagcaggtcgttgccgtgctgcttggggcaggcgtaaaagaaaaagcccaggaattagtt gcctgcggtgcagataaagttgttgtcgtagatgacattatgctggcagattatgtgacagagccatacacaaaggcactgaca gctgtgattaacgcaaaagatcctgagatcgtgctttatggtgcatcttccatcggacgtgaccttgccccaagagtatctgcaag gatccatactggattgacagcggactgtactgcacttgccattgatgaagagacaaagctcctcatgatgacccgtccggcattt ggcggaaatatcatggcgaccatcgtgtgtgaagacttccgtcctcagatggcaacagtccgtccaggagtaatgaaagcctt ggaaaacgatgcatccagaagcggtgaagtggaagagttcaaagtagaattctctgatgcagacatgaatgtaaaagtccgt gaaactgtaaagacagcacataaatctgtagacattacagaggctaagatccttgtttccggtgggcgcggaatcggaagcg ctgacaagttcaaggtgttagaggaactggcggatgtcatggaaggtgaagtgtcttcttccagagcatgtgtagattccggatg |
| gatcagtgctgaccgccaggtaggacagacaggaaagactgtgcgtccggaattatatctggcctgcggcatctcaggagcg atccagcatgcagccggtatggagaactcagagtttatcgtagccatcaaccgtgatgaggatgcgccgatctttgatattgcag acctcggcatcgttggagacttaaataccatcgtaccgaaacttacagaagctgtgaaggcggtaaaaggaaaataa |
| Amino acid sequence (SEQ ID NO:38) |
| MSRDVYVFAEQRDGELQKVGIELIGKARELADDLGQQVVAVLLGAGVKEKAQELVACGAD KVVVVDDIMLADYVTEPYTKALTAVINAKDPEIVLYGASSIGRDLAPRVSARIHTGLTADCTA LAIDEETKLLMMTRPAFGGNIMATIVCEDFRPQMATVRPGVMKALENDASRSGEVEEFKV EFSDADMNVKVRETVKTAHKSVDITEAKILVSGGRGIGSADKFKVLEELADVMEGEVSSSR ACVDSGWISADRQVGQTGKTVRPELYLACGISGAIQHAAGMENSEFIVAINRDEDAPIFDIA DLGIVGDLNTIVPKLTEAVKAVKGK |

| ***Inosose isomerase* - *AR1Y2_1058*** |
|---|
| Nucleotide sequence (SEQ ID NO:39) |
| atgaaaatcggatttaacgaagcaactgcactggaatgtaaaggacagtctctgatggctgacttagaagcgtgtgaaaaata cggatttgattacatcgagatcagatttgactgtgtaaaggattatttgaaagaacacacactggaagaactggcggactggttt aaaaaccatcatttaaaaccatgggcttataataccttgatcttttttaaccagagagacgaagccggagaaaaagagattgac gaagagactgattttatcattgaagtgtgcaaggccatcggcatgaagatgctgatcactgttccgtcctttgatgtaaaggataa gagcatcacagagatcaaagaggaagcagtagaacgtctccgttatttatccgataaggtgggagaagatattaagatatcac tggaattctgcggggcgccgaactgctccatcaaccagtttgggacagcttacgatgtagtgaaagcaacagaccgtgacaat gtaggggtaacagtggataccttccatttccatgagatgtgttctaagttagaagatttagaagcagcggacggaaagaagatc tttgcttaccatctgaatgactgtgaggatctgcctctgggatcatgcggtgatgataaacgcctgtggccgggagaaggtgtggt agatcatgcaggaatcgcagctgccttaaagaagatcggattcgacggggtatgtaccattgaagaattccgtccagaatatta tgaaatgtcccatgacgagaatgtgaaaaagtctgctgaagtgacaagggatttcgtagcaaaatactttgcataa |
| Amino acid sequence (SEQ ID NO:40) |
| MKIGFNEATALECKGQSLMADLEACEKYGFDYIEIRFDCVKDYLKEHTLEELADWFKNHHL KPWAYNTLIFFNQRDEAGEKEIDEETDFIIEVCKAIGMKMLITVPSFDVKDKSITEIKEEAVER LRYLSDKVGEDIKISLEFCGAPNCSINQFGTAYDVVKATDRDNVGVTVDTFHFHEMCSKLE DLEAADGKKIFAYHLNDCEDLPLGSCGDDKRLWPGEGVVDHAGIAAALKKIGFDGVCTIEE FRPEYYEMSHDENVKKSAEVTRDFVAKYFA |

| ***Lactate dehydrogenase* - *AR1Y2_2463*** |
|---|
| Nucleotide sequence (SEQ ID NO:41) |
| atgacatataaaatgagaaaaatagtgatcataggggccggacatgtgggaaccatgtgcggcctgagtcttatgtaccgcgg agaagtggacgaattggtattcatagacatcgataaggaaaaggcatacagccaggcgctggaccttagtgatgccgtctcct tcctgccccggcagatcacggtgcgcaccggggattatgaagatgtggaggacgctgacatcatcgttatggccgctggtgttt ccaggctgcccggtcagacaaggctggatatgctggacgattccatccgcatcatgaaagaagtcaccagccatttaaaaga taagaagattcagggcatcctcatcagtatctccaatcctgccgacattgtggcggattatctgagaaaacaactgggccttccg |
| agaaaccgctgcttcagcaccggtacttccctggactcctgccgtctcagaaggattctttcccaaagaatgcacttagaccgg aattccattcaggcctttgtcatgggagaacacggagattcccaaatgatcccgttttctgcggtacatatagccgggaaaccatt gcttcagtggatgcaagatgaaccggaacggtacggaagtctcgatctttcacagattgaaacggagaccgccaacgccgg ccatgaagtgatcgaaggaaagggcagcacagagtttggtatcggcatcgccctgtccgagatcgtccagtccattttccacg attcaaaaaaggtccttcccgcttcgcctctgctggaaggagaatatggacagcatggcgttcacgccggtgtcccctgcgtgat tggaaaagccggaattgaaaagatcattgagatccctctcacagaccgggagcaagagaaatttgaccattcctgcgatgtg atcagaacgtttattcaagatctctga |
| Amino acid sequence (SEQ ID NO:42) |
| MTYKMRKIVIIGAGHVGTMCGLSLMYRGEVDELVFIDIDKEKAYSQALDLSDAVSFLPRQIT VRTGDYEDVEDADIIVMAAGVSRLPGQTRLDMLDDSIRIMKEVTSHLKDKKIQGILISISNPA DIVADYLRKQLGLPRNRCFSTGTSLDSCRLRRILSQRMHLDRNSIQAFVMGEHGDSQMIP FSAVHIAGKPLLQWMQDEPERYGSLDLSQIETETANAGHEVIEGKGSTEFGIGIALSEIVQSI FHDSKKVLPASPLLEGEYGQHGVHAGVPCVIGKAGIEKIIEIPLTDREQEKFDHSCDVIRTFI QDL |

| ***Acetaldehyde dehydrogenase* - *AR1Y2_1414*** |
|---|
| Nucleotide sequence (SEQ ID NO:43) |
| atgggaagatttacattacctagagatttgtactttggaaaaggatcacttgagacactgaagacgctggacggaaaaaaagc tgttttagtcatcggcggcggatctatgaaacgttttggatttgtagaccaggttgttgattatttaaaagaagcaggcatagagac aaaattaattgagggagtagagccggacccttctgttgagaccgtatttaaaggtgccgagattatgagagagtttgaaccgga ctggatcgtggcaatgggcggaggatctccgatcgatgctgcaaaagctatgtggatcttctatgaacatccggaaaccacattt gacgatgtaaaagacccattcacagttccaaaactcagaaacaaagcaagattccttgcaatcccttcaacttcaggaacagc tacggaagtgacggcatttgctgttatcacggactacagttcaggcattaaatatccgctggcagactttgagatcacaccggat gttgccattgtagaccctgcactggcagagacgatgcctccgaaactgacctcttatacaggaatggatgcactgacccatgcg attgaagcatatgttgccggactgcattcaccattttcagatcctttggcaatcgatgccatcgagatggtatttgacaacttaaaag cttcctatgagggagacatgaccgcacgtgaaaatatgcactatgcacaatgtcttgcaggaatgtcattctctaatgcactgtta ggaattgtacattctttagcgcataagacaggagcagtttttgatacaggacatatcacacacggatgtgcgaacgctatctatct tccgtatgtgatccagtttaatgcaaaggtatgcgaagaccgctatgcagccattgcaagaaagatcggcctccctggaaattc cgataaagcactgacactttctttgattgaagcaatccgggatatgaacaagaagctgaacattgtttccaacctgaaagatttc ggaatcaatgaagaagaattcaaccagaagaagaggatgatcgcggagcgtgctgtggcagacgcatgtacaggatctaat cctagagaaacctctgtagatgacatggaaaaagtgttagagtgcatctactacggaaaagacgtaatattctaa |
| Amino acid sequence (SEQ ID NO:44) |
| MGRFTLPRDLYFGKGSLETLKTLDGKKAVLVIGGGSMKRFGFVDQVVDYLKEAGIETKLIE GVEPDPSVETVFKGAEIMREFEPDWIVAMGGGSPIDAAKAMWIFYEHPETTFDDVKDPFT VPKLRNKARFLAIPSTSGTATEVTAFAVITDYSSGIKYPLADFEITPDVAIVDPALAETMPPKL TSYTGMDALTHAIEAYVAGLHSPFSDPLAIDAIEMVFDNLKASYEGDMTARENMHYAQCLA GMSFSNALLGIVHSLAHKTGAVFDTGHITHGCANAIYLPYVIQFNAKVCEDRYAAIARKIGLP |
| GNSDKALTLSLIEAIRDMNKKLNIVSNLKDFGINEEEFNQKKRMIAERAVADACTGSNPRET SVDDMEKVLECIYYGKDVIF |

| ***Alcohol dehydrogenase* - *AR1Y2_2121*** |
|---|
| Nucleotide sequence (SEQ ID NO:45) |
| atgaaaataagtccgaaagtccgtgaatttttgtgccttaccgcacatcctcagggctgcagaaaaaatgttgaagatcagatta catatgtaaaaaaacacagcggtgttgagaatgggccgaaaaaggtcctggtcatcggctcatccacgggatatggcctggc atcaaggatcaccgctgcattcgggtgcggagcggataccttgggtgtcatgtttgagagaccggcggccggtaaacgtaccg ccactgccggatggtataatacagctgcatttgaacaggcggcggccagagaaggcttatatgcgaagacggtcaacgcag atgcattttccagggaagtaaaagaccaggtcatccaactgatcaagaaggatttcggtaaggttgacgctgtcatttacagtct ggctgcaccgagaagaacgacagaggacggcacggtttatcagtctgtcttaaagacaacaggggaagattttacaaataa gaacctgaaccttaaagataataccatcagtgagaaaaccatcgaggctgccacagaggaagaagtgaaagctaccgtga aggtcatgggcggggaggactggaaggcatggatccaggcgcttaccgatgcggatgccattgaaaagggcgcagtcacct tcgcatattcttatatcggacctaagcttacatatcctatctattttgagggaaccatcggtgccgcgaagaagcatcttcataaga cggcgggtgagatcaatgaggaattctcaaaggatggaatcaaagcatacatctccgtcaataaggggctggtgacacagg ccagttctgccattccgatcgtaccgctttatatggctgtgctgtaccgcgttatgaaagaaaagggacttcatgaaggctgtatcg aacagatcgtaaggctgttttctgaaaaggctgtctttggacaggccaagacagatgaacagggtctgatccgtctggatgact gggaactgcgcccggaagtccaggaagaagtggaacgccgctgggagaaggtctgcaccgaaaatgtccaggagtattct gatattgacggatactgggatgatttctaccaaatgttcggcttccgtatggatggagtggactacgaggaggatcttgacccttct gtggatatcccaagcatttccggccaagaatag |
| Amino acid sequence (SEQ ID NO:46) |
| MKISPKVREFLCLTAHPQGCRKNVEDQITYVKKHSGVENGPKKVLVIGSSTGYGLASRITA AFGCGADTLGVMFERPAAGKRTATAGWYNTAAFEQAAAREGLYAKTVNADAFSREVKD QVIQLIKKDFGKVDAVIYSLAAPRRTTEDGTVYQSVLKTTGEDFTNKNLNLKDNTISEKTIEA ATEEEVKATVKVMGGEDWKAWIQALTDADAIEKGAVTFAYSYIGPKLTYPIYFEGTIGAAKK HLHKTAGEINEEFSKDGIKAYISVNKGLVTQASSAIPIVPLYMAVLYRVMKEKGLHEGCIEQI VRLFSEKAVFGQAKTDEQGLIRLDDWELRPEVQEEVERRWEKVCTENVQEYSDIDGYWD DFYQMFGFRMDGVDYEEDLDPSVDIPSISGQE |

| ***Pyruvate kinase*** - ***AR1Y2_2344*** |
|---|
| Nucleotide sequence (SEQ ID NO:47) |
| atgctgacaaaaaaaacaaagattgtttgcacgatggggcctgcgacagatgatgatgcggtgctcaaacagctgatgctgg aaggaatggatatcgcgaggctgaatttctctcacggtgaccatgaggaacagcttggaaggatcaagagaattaaaagattc cgtgaagaactgaatattccgatcgcgatcttactggatacaaaaggacctgagatccgtacaggacttctggagacagacgg agacgtggaattaaaagaaggaaatgaatttgttctgaccacaagggacatcaaaggaaatgattctatcgtagcaattacat acgaagagcttccgcaggatgtcacagaaggaaatacaatcctgatcgatgacggtctgatcgaactgagagtcaaagaga tcaaagacggaacagatattgtatgtgaaattgtaaacggcggacttttaggaagccgtaagggagtgaacgtaccgaacgt |
| gcgcgtaaatcttccttccatcacagaaaaggataagagagacattgagttcggacttgaaaacggcattgattttatcgcggct tctttcatccgtaacgcagatgcagtcaatgagatcaaggacatcgtaaaagcccacaacatggaagtcggcgttatctccaa aattgaaaacgtggagggtgtggaaaatattgaatccatcatccaggcttctgacggaatcatggtagcccgtggagatttaggt gtggaaattcccgcagaggaagttccgtttatgcagaaagcaatcattaagaaatgcaacaatgcatttaagccagtcatcac agctacacagatgttagactctatgatcagaaacccacgtccgacaagagcggaagtaacagacgttgccaatgctatttatg acggaaccgatgcgattatgctttctggagaaaccgcgaaggggaaataccctgtggaggccgtaaggatgatgaaccaga tcgccatctccacagaactgcatctggatacgaagatcaaagattttcgttccatctatgtaagccgtggaatctcagcagcggtt gcttattctgctgtacagactgctcataatattaacgcaaaatgtatcctggcgtccagtatgtccggatttacggcaaggatcgtat ctaagttcaaaccggatgcacagatcattggcttgtctccggatgaagcggttgtgcgtaagatgcagctttactggggagtgcg tccgtttaagtcacataaggcttcctctaccagagaacttttggatgaagctacggatatcgtactgaatgcagatcttgcacagg agaacgacatcctggtactgaccggaggaggtccggcagaccaccgtggaaaaggcgtgacaaacatgctgaaagtagta aaaatcggcgaataa |
| Amino acid sequence (SEQ ID NO:48) |
| MLTKKTKIVCTMGPATDDDAVLKQLMLEGMDIARLNFSHGDHEEQLGRIKRIKRFREELNIP IAILLDTKGPEIRTGLLETDGDVELKEGNEFVLTTRDIKGNDSIVAITYEELPQDVTEGNTILID DGLIELRVKEIKDGTDIVCEIVNGGLLGSRKGVNVPNVRVNLPSITEKDKRDIEFGLENGIDFI AASFIRNADAVNEIKDIVKAHNMEVGVISKIENVEGVENIESIIQASDGIMVARGDLGVEIPAE EVPFMQKAIIKKCNNAFKPVITATQMLDSMIRNPRPTRAEVTDVANAIYDGTDAIMLSGETA KGKYPVEAVRMMNQIAISTELHLDTKIKDFRSIYVSRGISAAVAYSAVQTAHNINAKCILASS MSGFTARIVSKFKPDAQIIGLSPDEAVVRKMQLYWGVRPFKSHKASSTRELLDEATDIVLN ADLAQENDILVLTGGGPADHRGKGVTNMLKVVKIGE |

Should there be an inconsistency between the sequences disclosed in the description and the sequences disclosed in the sequence listing, the sequences disclosed in the description are preferred. Alternatively, the sequences of the sequence listing may be used.

### EXPERIMENTAL SUPPORT

### Bacterium comprising myo-inositol to propionate pathway

The present inventors found that myo-inositol can serve as carbon and energy source for a bacterium according to the present disclosure, e.g. *Anaerostipes rhamnosivorans* as previously isolated from stool of a healthy infant (Bui et al 2014 International Journal of Systematic and Evolutionary Microbiology 64: 787-793).

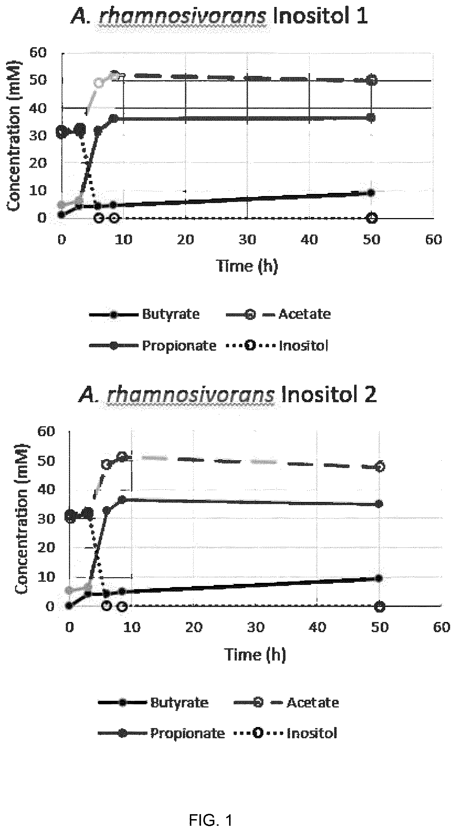

Figure 1 shows that this bacterium is able to ferment efficiently myo-inositol to propionate. The experiment was performed twice.

### Proteomics

A complete inositol fermentation pathway has been identified in the closed genome of *Anaerostipes rhamnosivorans,* which can thus be introduced in any bacterium of choice.

### Method for proteomics

To identify the proteins involved in the pathway, the protein abundances in cultures growing with two different substrates were examined with liquid chromatography-mass spectrometry/mass spectrometry.

*Anaerostipes rhamnosivorans* was grown in 4 replicates in 100 ml bicarbonate-buffered medium containing 40 mM myo-inositol or containing 40 mM rhamnose as carbon and energy sources.

Cells were collected in the exponential phase by centrifugation at 4700g at 4 °C for 20 min. Cell pellets were washed twice and suspended in 0.5 ml of 100 mM Tris-HCl (pH 8). Protein extraction and digestion were performed as described previously (Bui et al., 2015). After the digestion, the samples were kept at -20°C upon the analysis. The samples were analysed by injecting 4 µl sample over 0.10 * 250 mm ReproSil-Pur 120 C18-AQ 1.9 µm beads analytical column (prepared in-house) with an acetonitrile gradient at a flow of 0.5 ul/min with a Thermo EASY nanoLC1000. The gradient consisted of an increase from 9 to 34% acetonitrile in water with 1 ml/l formic acid in 50 minutes followed by a fast increase in the percentage acetonitrile to 80% (with 20% water and 1 ml/l formic acid in both the acetonitrile and the water) in 3 minutes as a column cleaning step. An electrospray potential of 3.5 kV was applied directly to the eluent via a stainless steel needle fitted into the waste line of the micro cross. Full scan positive mode FTMS spectra were measured between m/z 380 and 1400 on a Q-Exactive HFX (Thermo electron, San Jose, CA, USA) in the Orbitrap at high resolution (60000). MS and MSMS AGC targets were set to 3.10⁶, 50000 respectively or maximum ion injection times of 50 ms (MS) and 25 ms (MSMS) were used. HCD fragmented (Isolation width 1.2 m/z, 24% normalized collision energy) MSMS scans of the 25 most abundant 2-5+ charged peaks in the MS scan were recorded in data dependent mode (Threshold 1.2e5, 15 s exclusion duration for the selected m/z +/- 10 ppm). LCMS runs with all MSMS spectra obtained were analysed with MaxQuant 1.6.3.4 (Cox *et al., 2008)* using the "Specific Trypsin/P" Digestion mode with maximally 2 missed cleavages and further default settings for the Andromeda search engine (First search 20 ppm peptide tolerance, main search 4.5 ppm tolerance, MSMS fragment match tolerance of 0.5 Da, Propionamide (C) set as a fixed modification, while variable modifications were set for Protein N-terminal Acetylation and M oxidation which were completed by non-default settings for de-amidation of N and Q, the maximum number of modifications per peptide was 5 (*Cox et al., 2011). Anaerostipes rhamnosivorans* database from NCBI (CP040058) was used together with a contaminants database that contains sequences of common contaminants like Trypsins (P00760, bovin and P00761, porcin) and human keratins (Keratin K22E (P35908), Keratin K1C9 (P35527), Keratin K2C1 (P04264) and Keratin K1Cl (P35527)). The "label-free quantification" as well as the "match between runs" options were enabled. De-amidated peptides were allowed to be used for protein quantification and all other quantification settings were kept default.

Filtering and further bioinformatic analysis of the MaxQuant/Andromeda workflow output and the analysis of the abundances of the identified proteins were performed with the Perseus 1.6.2.1 module (available at the MaxQuant suite). Accepted were peptides and proteins with a false discovery rate (FDR) of less than 1% and proteins with at least 2 identified peptides of which at least one should be unique and at least one should be unmodified. Reversed hits were deleted from the MaxQuant result table as well as all results showing a normalised label free quantitation intensity (LFQ) value of 0 for all inositol and rhamnose injections. The normal logarithm was taken from protein LFQ MS1 intensities as obtained from MaxQuant. Zero "Log LFQ" values were replaced by a value of 5.9 (A value slightly lower than the lowest measured value) to make sensible ratio calculations possible. Relative protein quantitation of inositol to rhamnose was done with Perseus by applying a two sample Ttest using the "LFQ intensity" columns obtained with FDR set to 0.05 and S0 set to 1. nLC-MSMS system quality was checked with PTXQC (Bielow et al., 2016) using the MaxQuant result files.

### References:

Bui TPN, Ritari J, Boeren S, de Waard P, Plugge CM, de Vos WM. (2015). Production of butyrate from lysine and the Amadori product fructoselysine by a human gut commensal. Nature Communication 6*.*
Cox, J.; Mann, M. (2008). MaxQuant enables high peptide identification rates, individualized p.p.b.-range mass accuracies and proteome-wide protein quantification. Nature Biotechnology 26(12):1367-1372.
Cox, N. Neuhauser, A. Michalski, R. A. Scheltema, J. V. Olsen and M. Mann (2011). Andromeda: A Peptide Search Engine Integrated into the MaxQuant. Environment.Journal of Proteome Research. 10(4): 1794-1805.
Bielow, C., G. Mastrobuoni and S. Kempa (2016). Proteomics Quality Control: Quality Control Software for MaxQuant Results. Journal of Proteome Research 15(3): 777-787.

### Beneficial effect

It is known that dietary myo-inositol supplementation can reduce insulin resistance. The mechanism through which this occurs, however, is unknown. The present inventors considered that myo-inositol may have an indirect beneficial effect.

The present inventors envisaged that it is rather the metabolic conversion of myo-inositol that is beneficial to the host, rather than the myo-inositol itself. Hence, it is of interest to note that the production of butyrate and, notably, propionate, by *A. rhamnosivorans* is certainly beneficial for the patient (Hamer et al 2008 Pharmacology & Therapeutics 27: 104-119; Hosseini et al 2011 Nutrition Reviews 69: 245-258).

Hence, the supplementation of the patient with a bacterium according to the present disclosure, e.g. *A. rhamnisovorans* or alternatively both myo-inositol and *A. rhamnosivorans* provides a direct or synergistic effect on improved insulin resistance via additional propionate and butyrate production.

### Treatment

Patients are treated as indicated below, according to the following 3 treatment arms:
Treatment 1: 6 weeks, twice daily empty supplement.
Treatment 2: 6 weeks, twice daily supplement with 10⁸ living cells of a bacterium according to the present disclosure, i.e. *A. rhamnisovorans.*
Treatment 3: 6 weeks, twice daily supplement with 10⁸ living cells of a bacterium according to the present disclosure, i.e. *A. rhamnisovorans* in combination with 10 mg myo-inositol.

| *No. of patients* | *Condition* | *Effect treatment 1 (patient 1)* | *Effect treatment 2 (patient 2)* | *Effect treatment 3 (patient 3)* |
|---|---|---|---|---|
| 3 | Insulin resistance | No effect | Reduction of Insulin resistance with 10% | Reduction of Insulin resistance with 12.5% |

It is expected that results similar to the putative effects as shown in the table above can be obtained with larger patient cohorts.

### Other host bacteria

Another host bacterium, i.e. *Escherichia coli* is transfected according to standard methods with a gene set according to the present invention and comprising myo-inositol 2-dehydrogenase, inosose dehydratase, epi-inositol hydrolase, 5-deoxy-glucuronate isomerase, 5-keto-2-deoxygluconokinase, 5-keto-2-deoxy-D-gluconate-6 phosphate aldolase, glyceraldehyde-3-phosphate ketol-isomerase, glyceraldehyde-3-phosphate dehydrogenase, phosphoglycerate kinase, phosphoglycerate mutase, enolase, pyruvate kinase, pyruvate-flavodoxin oxidoreductase, 3-oxoacid CoA transferase. acyl-CoA dehydrogenase, enoyl-CoA hydratase, acryloyl-CoA dehydrogenase/Etf, and inosose isomerase. It is found that the host bacterium is capable of converting (myo)inositol to propionate under anaerobic conditions.

## Claims

1. Bacterium comprising a gene set that allows said bacterium to convert inositol to propionic acid or a salt or ester thereof, for use in preventing and/or treating metabolic syndrome, insulin resistance or insulin resistance-related condition, wherein the gene set comprises:
a gene encoding myo-inositol 2-dehydrogenase and having at least 70% sequence identity with SEQ ID NO:1 and/or SEQ ID NO:3,
a gene encoding inosose dehydratase and having at least 70% sequence identity with SEQ ID NO:5,
a gene encoding epi-inositol hydrolase and having at least 70% sequence identity with SEQ ID NO:7,
a gene encoding 5-deoxy-glucuronate isomerase and having at least 70% sequence identity with SEQ ID NO:9,
a gene encoding 5-keto-2-deoxygluconokinase and having at least 70% sequence identity with SEQ ID NO:11,
a gene encoding 5-keto-2-deoxy-D-gluconate-6 phosphate aldolase and having at least 70% sequence identity with SEQ ID NO:13,
a gene encoding glyceraldehyde-3-phosphate ketol-isomerase and having at least 70% sequence identity with SEQ ID NO:15,
a gene encoding glyceraldehyde-3-phosphate dehydrogenase and having at least 70% sequence identity with SEQ ID NO:17,
a gene encoding phosphoglycerate kinase and having at least 70% sequence identity with SEQ ID NO:19,
a gene encoding phosphoglycerate mutase and having at least 70% sequence identity with SEQ ID NO:21,
a gene encoding enolase and having at least 70% sequence identity with SEQ ID NO:23,
a gene encoding pyruvate kinase and having at least 70% sequence identity with SEQ ID NO:47,
a gene encoding pyruvate-flavodoxin oxidoreductase and having at least 70% sequence identity with SEQ ID NO:25,
a gene encoding 3-oxoacid CoA transferase and having at least 70% sequence identity with SEQ ID NO:27,
a gene encoding acyl-CoA dehydrogenase and having at least 70% sequence identity with SEQ ID NO:29,
a gene encoding enoyl-CoA hydratase and having at least 70% sequence identity with SEQ ID NO:31,
a gene encoding acryloyl-CoA dehydrogenase/Etf and having at least 70% sequence identity with SEQ ID NO:33, SEQ ID NO:35, and/or SEQ ID NO:37, and
a gene encoding inosose isomerase and having at least 70% sequence identity with SEQ ID NO:39.

2. Bacterium for use according to claim 1, wherein at least one of said genes encoding the proteins: myo-inositol 2-dehydrogenase, inosose dehydratase, epi-inositol hydrolase, 5-deoxy-glucuronate isomerase, 5-keto-2-deoxygluconokinase, 5-keto-2-deoxy-D-gluconate-6 phosphate aldolase, glyceraldehyde-3-phosphate keto-isomerase, glyceraldehyde-3-phosphate dehydrogenase, phosphoglycerate kinase, phosphoglycerate mutase, enolase, pyruvate kinase, pyruvate-flavodoxin oxidoreductase, 3-oxoacid CoA transferase. acyl-CoA dehydrogenase, enoyl-CoA hydratase, acryloyl-CoA dehydrogenase/Etf, and inosose isomerase, is overexpressed when the bacterium is grown in the presence of inositol as compared to when the bacterium is grown in the absence of inositol.

3. Bacterium for use according to any one of the previous claims, wherein the use is in preventing and/or treating insulin resistance or insulin resistance-related condition.

4. Bacterium for use according to any one of the previous claims, wherein the insulin resistance-related condition is chosen from dyslipidemia, type 2 diabetes mellitus and insulin-resistance in endocrine disease.

5. Bacterium for use according to any one of the previous claims, wherein the bacterium is a human intestinal isolate bacterium.

6. Bacterium for use according to any one of the previous claims, wherein the bacterium is deposited as CBS 140182.

7. Bacterium for use according to any one of the previous claims, wherein the bacterium is comprised in a composition further comprising a physiologically acceptable carrier.

8. Bacterium for use according to claim 7, wherein the composition is a food composition, preferably a dairy product, more preferably a fermented dairy product, preferably a yogurt or a yogurt drink.

9. Bacterium for use according to claim 7, wherein the composition is a pharmaceutical composition, preferably in solid dosage form, preferably a capsule, a tablet, or a powder.

10. Bacterium for use according to any one of claims 7-9, wherein in the composition the bacterium is present in lyophilized or microencapsulated form.

11. Bacterium for use according to any one of the previous claims, wherein the bacterium is present in an amount within the range of between 10⁶ to 10¹⁵ colony forming units (CFU)

12. Bacterium for use according to any one of the previous claims, wherein the bacterium is administered separately, sequentially or simultaneously with inositol.

13. Bacterium for use according to any one of the previous claims for use as a probiotic.

## Patentansprüche

1. Bakterium, das einen Gensatz umfasst, der es dem Bakterium ermöglicht, Inositol in Propionsäure oder ein Salz oder einen Ester davon umzuwandeln, zur Verwendung bei der Prävention und/oder Behandlung des metabolischen Syndroms, der Insulinresistenz oder eines mit der Insulinresistenz zusammenhängenden Zustands, wobei der Gensatz umfasst:
ein Gen, das für Myoinositol-2-dehydrogenase codiert und mindestens 70 % Sequenzidentität mit SEQ ID NO:1 und/oder SEQ ID NO:3 aufweist,
ein Gen, das für Inososedehydratase codiert und mindestens 70 % Sequenzidentität mit SEQ ID NO: 5 aufweist,
ein Gen, das für Epiinsitolhydrolase codiert und mindestens 70 % Sequenzidentität mit SEQ ID NO:7 aufweist,
ein Gen, das für 5-Desoxyglucuronatisomerase codiert und mindestens 70 % Sequenzidentität mit SEQ ID NO:9 aufweist,
ein Gen, das für 5-Keto-2-desoxygluconokinase codiert und mindestens 70 % Sequenzidentität mit SEQ ID NO:11 aufweist,
ein Gen, das für 5-Keto-2-desoxy-D-gluconat-6-phosphataldolase codiert und mindestens 70 % Sequenzidentität mit SEQ ID NO:13 aufweist,
ein Gen, das für Glyceraldehyd-3-phosphatketolisomerase codiert und mindestens 70% Sequenzidentität mit SEQ ID NO:15 aufweist,
ein Gen, das für Glyceraldehyd-3-phosphatdehydrogenase codiert und mindestens 70 % Sequenzidentität mit SEQ ID NO:17 aufweist,
ein Gen, das für Phosphoglyceratkinase codiert und mindestens 70 % Sequenzidentität mit SEQ ID NO:19 aufweist,
ein Gen, das für Phosphoglyceratmutase codiert und mindestens 70 % Sequenzidentität mit SEQ ID NO:21 aufweist,
ein Gen, das für Enolase codiert und mindestens 70 % Sequenzidentität mit SEQ ID NO:23 aufweist,
ein Gen, das für Pyruvatkinase codiert und mindestens 70 % Sequenzidentität mit SEQ ID NO:47 aufweist,
ein Gen, das für Pyruvatflavodoxinoxidoreduktase codiert und mindestens 70 % Sequenzidentität mit SEQ ID NO:25 aufweist,
ein Gen, das für 3-Oxosäure-CoA-transferase codiert und mindestens 70 % Sequenzidentität mit SEQ ID NO:27 aufweist,
ein Gen, das für Acyl-CoA-dehydrogenase codiert und mindestens 70 % Sequenzidentität mit SEQ ID NO:29 aufweist,
ein Gen, das für Enoyl-CoA-Hydratase codiert und mindestens 70 % Sequenzidentität mit SEQ ID NO:31 aufweist,
ein Gen, das für Acryloyl-CoA-Dehydrogenase/Etf codiert und mindestens 70 % Sequenzidentität mit SEQ ID NO:33, SEQ ID NO:35 und/oder SEQ ID NO:37 aufweist, und
ein Gen, das für Inososeisomerase codiert und mindestens 70 % Sequenzidentität mit SEQ ID NO:39 aufweist.

2. Bakterium zur Verwendung nach Anspruch 1, wobei mindestens eines der Gene, die für die Proteine: Myoinositol-2-dehydrogenase, Inososedehydratase, Epiinositolhydrolase, 5-Desoxyglucuronatisomerase, 5-Keto-2-desoxygluconokinase, 5-Keto-2-desoxy-D-gluconat-6-phosphataldolase, Glyceraldehyd-3-phosphatketoisomerase, Glyceraldehyd-3-phosphatdehydrogenase, Phosphoglyceratkinase, Phosphoglyceratmutase, Enolase, Pyruvatkinase, Pyruvatflavodoxinoxidoreduktase, 3-Oxosäure-CoA-transferase. Acyl-CoA-dehydrogenase, Enoyl-CoA-hydratase, Acryloyl-CoA-dehydrogenase/Etf und Inososeisomerase codieren, überexprimiert wird, wenn das Bakterium in Anwesenheit von Inositol gezüchtet wird, verglichen mit dem Bakterium, das in Abwesenheit von Inositol gezüchtet wird.

3. Bakterium zur Verwendung nach einem der vorhergehenden Ansprüche, wobei die Verwendung in der Prävention und/oder Behandlung von Insulinresistenz oder einem mit Insulinresistenz zusammenhängenden Zustand besteht.

4. Bakterium zur Verwendung nach einem der vorhergehenden Ansprüche, wobei der mit der Insulinresistenz zusammenhängende Zustand ausgewählt ist aus Dyslipidämie, Diabetes mellitus Typ 2 und Insulinresistenz bei endokriner Erkrankung.

5. Bakterium zur Verwendung nach einem der vorhergehenden Ansprüche, wobei das Bakterium ein menschliches Darmisolatbakterium ist.

6. Bakterium zur Verwendung nach einem der vorhergehenden Ansprüche, wobei das Bakterium als CBS 140182 hinterlegt ist.

7. Bakterium zur Verwendung nach einem der vorhergehenden Ansprüche, wobei das Bakterium in einer Zusammensetzung enthalten ist, die zudem einen physiologisch akzeptablen Träger umfasst.

8. Bakterium zur Verwendung nach Anspruch 7, wobei die Zusammensetzung eine Lebensmittelzusammensetzung ist, vorzugsweise ein Milchprodukt, stärker bevorzugt ein fermentiertes Milchprodukt, vorzugsweise ein Joghurt oder ein Joghurtgetränk.

9. Bakterium zur Verwendung nach Anspruch 7, wobei die Zusammensetzung eine pharmazeutische Zusammensetzung ist, vorzugsweise in fester Dosierungsform, vorzugsweise eine Kapsel, eine Tablette oder ein Pulver.

10. Bakterium zur Verwendung nach einem der Ansprüche 7 bis 9, wobei das Bakterium in der Zusammensetzung in lyophilisierter oder mikroverkapselter Form vorliegt.

11. Bakterium zur Verwendung nach einem der vorhergehenden Ansprüche, wobei das Bakterium in einer Menge im Bereich von 10⁶ bis 10¹⁵ koloniebildenden Einheiten (colony forming units, CFU) vorhanden ist.

12. Bakterium zur Verwendung nach einem der vorhergehenden Ansprüche, wobei das Bakterium getrennt, nacheinander oder gleichzeitig mit Inositol verabreicht wird.

13. Bakterium zur Verwendung nach einem der vorhergehenden Ansprüche zur Verwendung als Probiotikum.

## Revendications

1. Bactérie comprenant un ensemble de gènes qui permet à ladite bactérie de convertir l'inositol en acide propionique ou un sel ou un ester de celui-ci, destinée à être utilisée dans la prévention et/ou le traitement du syndrome métabolique, de la résistance à l'insuline ou d'une affection liée à la résistance à l'insuline, dans laquelle l'ensemble de gènes comprend :
un gène codant pour la myo-inositol 2-déshydrogénase et présentant au moins 70 % d'identité de séquence avec SEQ ID NO : 1 et/ou SEQ ID NO : 3,
un gène codant pour l'inosose déshydratase et présentant au moins 70 % d'identité de séquence avec SEQ ID NO : 5,
un gène codant pour l'épi-inositol hydrolase et présentant au moins 70 % d'identité de séquence avec SEQ ID NO : 7,
un gène codant pour la 5-désoxy-glucuronate isomérase et présentant au moins 70 % d'identité de séquence avec SEQ ID NO : 9,
un gène codant pour la 5-céto-2-désoxygluconokinase et présentant au moins 70 % d'identité de séquence avec SEQ ID NO : 11,
un gène codant pour la 5-céto-2-désoxy-D-gluconate-6 phosphate aldolase et présentant au moins 70 % d'identité de séquence avec SEQ ID NO : 13,
un gène codant pour la glycéraldéhyde-3-phosphate cétol-isomérase et présentant au moins 70 % d'identité de séquence avec SEQ ID NO : 15,
un gène codant pour la glycéraldéhyde-3-phosphate déshydrogénase et présentant au moins 70 % d'identité de séquence avec SEQ ID NO : 17,
un gène codant pour la phosphoglycérate kinase et présentant au moins 70 % d'identité de séquence avec SEQ ID NO : 19,
un gène codant pour la phosphoglycérate mutase et présentant au moins 70 % d'identité de séquence avec SEQ ID NO : 21,
un gène codant pour l'énolase et présentant au moins 70 % d'identité de séquence avec SEQ ID NO : 23,
un gène codant pour la pyruvate kinase et présentant au moins 70 % d'identité de séquence avec SEQ ID NO : 47,
un gène codant pour la pyruvate-flavodoxine oxydoréductase et présentant au moins 70 % d'identité de séquence avec SEQ ID NO : 25,
un gène codant pour la 3-oxoacide CoA transférase et présentant au moins 70 % d'identité de séquence avec SEQ ID NO : 27,
un gène codant pour l'acyl-CoA déshydrogénase et présentant au moins 70 % d'identité de séquence avec SEQ ID NO : 29,
un gène codant pour l'énoyl-CoA hydratase et présentant au moins 70 % d'identité de séquence avec SEQ ID NO : 31,
un gène codant pour l'acryloyl-CoA déshydrogénase/Etf et présentant au moins 70 % d'identité de séquence avec SEQ ID NO : 33, SEQ ID NO : 35 et/ou SEQ ID NO : 37, et
un gène codant pour l'inosose isomérase et présentant au moins 70 % d'identité de séquence avec SEQ ID NO : 39.

2. Bactérie destinée à être utilisée selon la revendication 1, dans laquelle au moins un desdits gènes codant pour les protéines : myo-inositol 2-déshydrogénase, inosose déshydratase, épi-inositol hydrolase, 5-désoxy-glucuronate isomérase, 5-céto-2-désoxygluconokinase, 5-céto-2-désoxy-D-gluconate-6 phosphate aldolase, glycéraldéhyde-3-phosphate céto-isomérase, glycéraldéhyde-3-phosphate déshydrogénase, phosphoglycérate kinase, phosphoglycérate mutase, énolase, pyruvate kinase, pyruvate-flavodoxine oxydoréductase, 3-oxoacide CoA transférase, acyl-CoA déshydrogénase, énoyl-CoA hydratase, acryloyl-CoA déshydrogénase/Etf et inosose isomérase, est surexprimé lorsque la bactérie est cultivée en présence d'inositol par rapport à la culture de la bactérie en l'absence d'inositol.

3. Bactérie destinée à être utilisée selon l'une quelconque des revendications précédentes, l'utilisation étant destinée à prévenir et/ou traiter la résistance à l'insuline ou une affection liée à la résistance à l'insuline.

4. Bactérie destinée à être utilisée selon l'une quelconque des revendications précédentes, l'affection liée à la résistance à l'insuline étant choisie parmi la dyslipidémie, le diabète sucré de type 2 et la résistance à l'insuline dans une maladie endocrinienne.

5. Bactérie destinée à être utilisée selon l'une quelconque des revendications précédentes, la bactérie étant une bactérie d'isolat intestinal humain.

6. Bactérie destinée à être utilisée selon l'une quelconque des revendications précédentes, la bactérie étant déposée sous CBS140182.

7. Bactérie destinée à être utilisée selon l'une quelconque des revendications précédentes, la bactérie étant comprise dans une composition comprenant en outre un véhicule physiologiquement acceptable.

8. Bactérie destinée à être utilisée selon la revendication 7, la composition étant une composition alimentaire, de préférence un produit laitier, plus préférablement un produit laitier fermenté, de préférence un yaourt ou une boisson au yaourt.

9. Bactérie destinée à être utilisée selon la revendication 7, la composition étant une composition pharmaceutique, de préférence sous une forme posologique solide, de préférence une capsule, un comprimé ou une poudre.

10. Bactérie destinée à être utilisée selon l'une quelconque des revendications 7 à 9, la bactérie étant présente dans la composition sous forme lyophilisée ou microencapsulée.

11. Bactérie destinée à être utilisée selon l'une quelconque des revendications précédentes, la bactérie étant présente en une quantité dans la plage de 10⁶ à 10¹⁵ unités formant des colonies (UFC).

12. Bactérie destinée à être utilisée selon l'une quelconque des revendications précédentes, la bactérie étant administrée séparément, séquentiellement ou simultanément avec l'inositol.

13. Bactérie destinée à être utilisée selon l'une quelconque des revendications précédentes pour utilisation en tant que probiotique.
